# EUROPEAN PATENT APPLICATION

(11) **EP 4 582 108 A1**
(43) Date of publication of application: **09.07.2025**
(21) Application number: 23860552.1
(22) Date of filing: 01.09.2023
(51) Int. Cl.: A61K 48/00, A61K 45/00, A61P 31/12, A61P 31/18, A61P 43/00, C07K 14/155, C12N 15/31, C12N 15/49

(54) **COMPOSITION FOR TREATING HIV INFECTION**

(30) Priority: 02.09.2022 JP 2022140218
(71) Applicant: National Institutes of Biomedical Innovation, Health and Nutrition, Ibaraki-shi, Osaka 567-0085 (JP)
(72) Inventor: YASUTOMI, Yasuhiro, Tsukuba-shi, Ibaraki 305-0843 (JP); OKAMURA, Tomotaka, Tsukuba-shi, Ibaraki 305-0843 (JP)
(74) Representative: Weickmann & Weickmann PartmbB
(86) International application number: PCT/JP2023/032141
(87) International publication number: WO 2024/048792

(57) **Abstract**

The present disclosure provides a composition for treating a viral infection. In one aspect, the present disclosure provides a composition for treating a viral infection in a subject, the composition containing a nucleic acid construct containing, in an operable manner, a nucleic acid sequence encoding an antigen protein contained in a virus belonging to the genus Lentivirus or a part thereof and a nucleic acid sequence encoding Ag85B protein. In one embodiment, the composition of the present disclosure is administered after the viral infection.

## Description

### Technical Field

The present disclosure provides a composition and a related technique for treating a viral infection. More specifically, the present disclosure provides a technique related to a nucleic acid construct containing, in an operable manner, a nucleic acid sequence encoding an antigen protein contained in a virus belonging to the genus Lentivirus or a part thereof and a nucleic acid sequence encoding Ag85B protein.

### Background Art

Despite the enormous resources invested in the development of effective human immunodeficiency virus (HIV) vaccines during the last 30 years, the object has not yet been achieved. Although antiretroviral therapy has resulted in a dramatic reduction in HIV-associated morbidity and mortality, the antiretroviral therapy cannot cure HIV (Non Patent Literature 1). There are two cases where remission of HIV is achieved by cell transplantation. When an effective vaccine is developed, the spread of HIV infection can be prevented. However, vaccine development has had little success during the past 30 years. The only successful HIV vaccine to date was evaluated in the RV144 clinical trial, which showed an overall efficacy of only 31% (Non Patent Literature 2).

### Citation List

### Non Patent Literature

Non Patent Literature 1: Cohen, M. S. et al. Prevention of HIV-1 infection with early antiretroviral therapy. N. Engl. J. Med. 365, 493-505 (2011).
Non Patent Literature 2: Rerks-Ngarm, S. et al. Vaccination with ALVAC and AIDSVAX to prevent HIV-1 infection in Thailand. N. Engl. J. Med. 361, 2209-2220 (2009).

### Summary of Invention

### Solution to Problem

The present inventors have found that a nucleic acid construct containing, in an operable manner, a nucleic acid sequence encoding an antigen protein contained in a virus belonging to the genus Lentivirus or a part thereof and a nucleic acid sequence encoding Ag85B protein, treats a viral infection in a subject, thereby completing the present invention.

Therefore, the present invention provides, for example, the following items.

### (Item 1)

A composition for treating a viral infection in a subject, the composition containing a nucleic acid construct containing, in an operable manner, a nucleic acid sequence encoding an antigen protein contained in a virus belonging to the genus Lentivirus or a part thereof and a nucleic acid sequence encoding Ag85B protein.

### (Item 2)

The composition according to the above item, characterized in that the composition is administered after the viral infection.

### (Item 3)

The composition according to any one of the above items, in which the treatment for the viral infection substantially eliminates the virus from the subject after the viral infection.

### (Item 4)

The composition according to any one of the above items, in which in the treatment for the viral infection, the composition is administered to substantially eliminate the virus from the subject after the viral infection.

### (Item 5)

The composition according to any one of the above items, in which the nucleic acid sequence encoding the antigen protein or part thereof is a nucleic acid sequence encoding an attenuated virus.

### (Item 6)

The composition according to any one of the above items, in which the attenuated virus is an nef-deleted attenuated virus.

### (Item 7)

The composition according to any one of the above items, in which the nucleic acid sequence encoding the Ag85B protein is incorporated into the nucleic acid sequence encoding the attenuated virus.

### (Item 8)

The composition according to any one of the above items, in which the nucleic acid sequence encoding the Ag85B protein is incorporated at a location of a deleted nef gene in a nucleic acid sequence encoding the nef-deleted attenuated virus.

### (Item 9)

The composition according to any one of the above items, in which the virus is an AIDS virus.

### (Item 10)

The composition according to any one of the above items, in which the virus is an attenuated virus of a virus that infects humans.

### (Item 11)

The composition according to any one of the above items, in which the virus is an attenuated virus of an AIDS virus selected from the group consisting of HIV, SIV, SHIV, and FIV.

### (Item 12)

The composition according to any one of the above items, in which the virus is an attenuated HIV.

### (Item 13)

The composition according to any one of the above items, in which the virus is an nef-deleted attenuated HIV.

### (Item 14)

The composition according to any one of the above items, in which the nucleic acid sequence encoding the antigen protein or part thereof includes a nucleic acid sequence at least 90% identical to a nucleic acid sequence containing nucleotides 1 to 9,633 and 10,612 to 11,022 of SEQ ID NO: 3 or a nucleic acid sequence containing nucleotides 1 to 8,786 and 9,765 to 15,182 of SEQ ID NO: 4.

### (Item 15)

The composition according to any one of the above items, in which the nucleic acid construct contains a nucleic acid sequence at least 90% identical to a nucleic acid sequence set forth in SEQ ID NO: 3 or 4.

### (Item 16)

The composition according to any one of the above items, in which the nucleic acid construct contains a nucleic acid sequence set forth in SEQ ID NO: 3 or 4.

### (Item 17)

The composition according to any one of the above items, characterized in that the nucleic acid construct is administered once.

### (Item 18)

The composition according to any one of the above items, characterized in that the nucleic acid construct is administered two or more times.

### (Item 18-A)

The composition according to any one of the above items, characterized in that the nucleic acid construct is administered after administration of an anti-HIV drug.

### (Item 18-B)

The composition according to any one of the above items, in which the anti-HIV drug is selected from the group consisting of a reverse transcriptase inhibitor, a protease inhibitor, an integrase inhibitor, and a CCR5 inhibitor, and preferably from tenofovir, emtricitabine, dolutegravir, zidovudine, lamivudine, sanilvudine, didanosine, abacavir, nevirapine, efavirenz, etravirine, rilpivirine, saquinavir, indinavir, nelfinavir, lopinavir/ritonavir combination, atazanavir, fosamprenavir, darunavir, ritonavir, raltegravir, elvitegravir, maraviroc, or any combination thereof.

### (Item 18-C)

The composition according to any one of the above items, characterized in that the nucleic acid construct is administered after administration of the anti-HIV drug and after discontinuing the administration of the HIV drug.

### (Item 18-D)

The composition according to any one of the above items, characterized in that the nucleic acid construct is administered two or more times after administration of the anti-HIV drug.

### (Item 18-E)

The composition according to any one of the above items, characterized in that the nucleic acid construct is administered three, four, five or more times after administration of the anti-HIV drug.

### (Item 18-F)

The composition according to any one of the above items, characterized in that two or more administrations of the nucleic acid construct are performed at an interval of at least one week or longer.

### (Item 18-G)

The composition according to any one of the above items, characterized in that two or more administrations of the nucleic acid construct are performed at an interval of one week to four weeks.

### (Item 18-H)

The composition according to any one of the above items, characterized in that two or more administrations of the nucleic acid construct are performed at an interval of one to three weeks.

### (Item 18-I)

The composition according to any one of the above items, characterized in that in two or more administrations of the nucleic acid construct, a plasma viral load of the patient is observed and the nucleic acid construct is administered again when a predetermined value is reached.

### (Item 18-J)

The composition according to any one of the above items, characterized in that the nucleic acid construct is administered at a dosage of 5.0 × 10⁵ TCID₅₀ or more or 1.0 × 10⁶ TCID₅₀ or more per administration.

### (Item 18-K)

The composition according to any one of the above items, characterized in that two or more administrations of the nucleic acid construct are performed at 5.0 × 10⁵ TCID₅₀ or more or 1.0 × 10⁶ TCID₅₀ or more per administration at an interval of one week to four weeks.

### (Item 19)

The composition according to any one of the above items, characterized in that the nucleic acid construct is administered once a week, once every two weeks, once every three weeks, once a month, or once every two months.

### (Item 19A)

The composition according to any one of the above items, characterized in that the nucleic acid construct is administered once a week, once every two weeks, once every three weeks, once a month, once every two months, or at a longer interval.

### (Item 20)

The composition according to any one of the above items, characterized in that the nucleic acid construct is administered at a dose of 1.0 × 10³ TCID₅₀ or more.

### (Item 21)

The composition according to any one of the above items, characterized in that the nucleic acid construct is administered at a dose of 1.0 × 10³ TCID₅₀ or more and less than 5.0 × 10⁴ TCID₅₀.

### (Item 22)

The composition according to any one of the above items, characterized in that the nucleic acid construct is administered once a week at a dose of 1.0 × 10³ TCID₅₀ or more and less than 5.0 × 10⁴ TCID₅₀.

### (Item 23)

The composition according to any one of the above items, characterized in that the nucleic acid construct is administered once at a dose of 1.0 × 10³ TCID₅₀ or more and less than 5.0 × 10⁴ TCID₅₀, and when a virus is detected in a body, the nucleic acid construct is further administered.

### (Item 24)

The composition according to any one of the above items, characterized in that the nucleic acid construct is administered at a dose of 5.0 × 10⁴ TCID₅₀ or more and 5.0 × 10⁶ TCID₅₀ or less.

### (Item 25)

The composition according to any one of the above items, characterized in that the nucleic acid construct is administered once at a dose of 5.0 × 10⁴ TCID₅₀ or more.

### (Item 26)

The composition according to any one of the above items, characterized in that an anti-HIV drug is already administered to the subject.

### (Item 27)

The composition according to any one of the above items, in which an anti-HIV drug is not yet administered to the subject, or the anti-HIV drug is discontinued at the start of administration of the composition.

### (Item 28)

The composition according to any one of the above items, characterized in that as the anti-HIV drug, an anti-HIV drug selected from the group consisting of a reverse transcriptase inhibitor, a protease inhibitor, an integrase inhibitor, and a CCR5 inhibitor is administered.

### (Item 29)

The composition according to any one of the above items, characterized in that as the anti-HIV drug, an anti-HIV drug selected from the group consisting of tenofovir, emtricitabine, dolutegravir, zidovudine, lamivudine, sanilvudine, didanosine, abacavir, nevirapine, efavirenz, etravirine, rilpivirine, saquinavir, indinavir, nelfinavir, lopinavir/ritonavir combination, atazanavir, fosamprenavir, darunavir, ritonavir, raltegravir, elvitegravir, maraviroc, or any combination thereof is administered.

### (Item 30)

The composition according to any one of the above items, in which the composition completely eliminates the AIDS virus in the subject.

### (Item 31)

The composition according to any one of the above items, in which the composition is not administered before the viral infection.

### (Item 32)

The composition according to any one of the above items, in which the subject is previously inoculated with a BCG vaccine.

### (Item 1A)

A method for treating a viral infection in a subject, the method including administering an effective amount of a nucleic acid construct containing, in an operable manner, a nucleic acid sequence encoding an antigen protein contained in a virus belonging to the genus Lentivirus or a part thereof and a nucleic acid sequence encoding Ag85B protein.

### (Item 2A)

The method according to the above item, characterized in that the nucleic acid construct is administered after the viral infection.

### (Item 3A)

The method according to any one of the above items, in which the treatment for the viral infection substantially eliminates the virus from the subject after the viral infection.

### (Item 4A)

The method according to any one of the above items, in which in the treatment for the viral infection, the nucleic acid construct is administered to substantially eliminate the virus from the subject after the viral infection.

### (Item 5A)

The method according to any one of the above items, in which the nucleic acid sequence encoding the antigen protein or part thereof is a nucleic acid sequence encoding an attenuated virus.

### (Item 6A)

The method according to any one of the above items, in which the attenuated virus is an nef-deleted attenuated virus.

### (Item 7A)

The method according to any one of the above items, in which the nucleic acid sequence encoding the Ag85B protein is incorporated into the nucleic acid sequence encoding the attenuated virus.

### (Item 8A)

The method according to any one of the above items, in which the nucleic acid sequence encoding the Ag85B protein is incorporated at a location of a deleted nef gene in a nucleic acid sequence encoding the nef-deleted attenuated virus.

### (Item 9A)

The method according to any one of the above items, in which the virus is an AIDS virus.

### (Item 10A)

The method according to any one of the above items, in which the virus is an attenuated virus of a virus that infects humans.

### (Item 11A)

The method according to any one of the above items, in which the virus is an attenuated virus of an AIDS virus selected from the group consisting of HIV, SIV, SHIV, and FIV.

### (Item 12A)

The method according to any one of the above items, in which the virus is an attenuated HIV.

### (Item 13A)

The method according to any one of the above items, in which the virus is an nef-deleted attenuated HIV.

### (Item 14A)

The method according to any one of the above items, in which the nucleic acid sequence encoding the antigen protein or part thereof includes a nucleic acid sequence at least 90% identical to a nucleic acid sequence containing nucleotides 1 to 9,633 and 10,612 to 11,022 of SEQ ID NO: 3 or a nucleic acid sequence containing nucleotides 1 to 8,786 and 9,765 to 15,182 of SEQ ID NO: 4.

### (Item 15A)

The method according to any one of the above items, in which the nucleic acid construct contains a nucleic acid sequence at least 90% identical to a nucleic acid sequence set forth in SEQ ID NO: 3 or 4.

### (Item 16A)

The method according to any one of the above items, in which the nucleic acid construct contains a nucleic acid sequence set forth in SEQ ID NO: 3 or 4.

### (Item 17A)

The method according to any one of the above items, characterized in that the nucleic acid construct is administered once.

### (Item 18A)

The method according to any one of the above items, characterized in that the nucleic acid construct is administered two or more times.

### (Item 18A-A)

The method according to any one of the above items, characterized in that the nucleic acid construct is administered after administration of an anti-HIV drug.

### (Item 18A-B)

The method according to any one of the above items, in which the anti-HIV drug is selected from the group consisting of a reverse transcriptase inhibitor, a protease inhibitor, an integrase inhibitor, and a CCR5 inhibitor, and preferably from tenofovir, emtricitabine, dolutegravir, zidovudine, lamivudine, sanilvudine, didanosine, abacavir, nevirapine, efavirenz, etravirine, rilpivirine, saquinavir, indinavir, nelfinavir, lopinavir/ritonavir combination, atazanavir, fosamprenavir, darunavir, ritonavir, raltegravir, elvitegravir, maraviroc, or any combination thereof.

### (Item 18A-C)

The method according to any one of the above items, characterized in that the nucleic acid construct is administered after administration of the anti-HIV drug and after discontinuing the administration of the HIV drug.

### (Item 18A-D)

The method according to any one of the above items, characterized in that the nucleic acid construct is administered two or more times after administration of the anti-HIV drug.

### (Item 18A-E)

The method according to any one of the above items, characterized in that the nucleic acid construct is administered three, four, five or more times after administration of the anti-HIV drug.

### (Item 18A-F)

The method according to any one of the above items, characterized in that two or more administrations of the nucleic acid construct are performed at an interval of at least one week or longer.

### (Item 18A-G)

The method according to any one of the above items, characterized in that two or more administrations of the nucleic acid construct are performed at an interval of one week to four weeks.

### (Item 18A-H)

The method according to any one of the above items, characterized in that two or more administrations of the nucleic acid construct are performed at an interval of one to three weeks.

### (Item 18A-I)

The method according to any one of the above items, characterized in that in two or more administrations of the nucleic acid construct, a plasma viral load of the patient is observed and the nucleic acid construct is administered again when a predetermined value is reached.

### (Item 18A-J)

The method according to any one of the above items, characterized in that the nucleic acid construct is administered at a dosage of 5.0 × 10⁵ TCID₅₀ or more or 1.0 × 10⁶ TCID₅₀ or more per administration.

### (Item 18A-K)

The method according to any one of the above items, characterized in that two or more administrations of the nucleic acid construct are performed at 5.0 × 10⁵ TCID₅₀ or more or 1.0 × 10⁶ TCID₅₀ or more per administration at an interval of one week to four weeks.

### (Item 19A)

The method according to any one of the above items, characterized in that the nucleic acid construct is administered once a week, once every two weeks, once every three weeks, once a month, or once every two months.

### (Item 19A)

The method according to any one of the above items, characterized in that the nucleic acid construct is administered once a week, once every two weeks, once every three weeks, once a month, once every two months, or at a longer interval.

### (Item 20A)

The method according to any one of the above items, characterized in that the nucleic acid construct is administered at a dose of 1.0 × 10³ TCID₅₀ or more.

### (Item 21A)

The method according to any one of the above items, characterized in that the nucleic acid construct is administered at a dose of 1.0 × 10³ TCID₅₀ or more and less than 5.0 × 10⁴ TCID₅₀.

### (Item 22A)

The method according to any one of the above items, characterized in that the nucleic acid construct is administered once a week at a dose of 1.0 × 10³ TCID₅₀ or more and less than 5.0 × 10⁴ TCID₅₀.

### (Item 23A)

The method according to any one of the above items, characterized in that the nucleic acid construct is administered once at a dose of 1.0 × 10³ TCID₅₀ or more and less than 5.0 × 10⁴ TCID₅₀, and when a virus is detected in a body, the nucleic acid construct is further administered.

### (Item 24A)

The method according to any one of the above items, characterized in that the nucleic acid construct is administered at a dose of 5.0 × 10⁴ TCID₅₀ or more and 5.0 × 10⁶ TCID₅₀ or less.

### (Item 25A)

The method according to any one of the above items, characterized in that the nucleic acid construct is administered once at a dose of 5.0 × 10⁴ TCID₅₀ or more.

### (Item 26A)

The method according to any one of the above items, characterized in that an anti-HIV drug is already administered to the subject.

### (Item 27A)

The method according to any one of the above items, in which an anti-HIV drug is not yet administered to the subject, or the anti-HIV drug is discontinued at the start of administration of the composition.

### (Item 28A)

The method according to any one of the above items, characterized in that as the anti-HIV drug, an anti-HIV drug selected from the group consisting of a reverse transcriptase inhibitor, a protease inhibitor, an integrase inhibitor, and a CCR5 inhibitor is administered.

### (Item 29A)

The method according to any one of the above items, characterized in that as the anti-HIV drug, an anti-HIV drug selected from the group consisting of tenofovir, emtricitabine, dolutegravir, zidovudine, lamivudine, sanilvudine, didanosine, abacavir, nevirapine, efavirenz, etravirine, rilpivirine, saquinavir, indinavir, nelfinavir, lopinavir/ritonavir combination, atazanavir, fosamprenavir, darunavir, ritonavir, raltegravir, elvitegravir, maraviroc, or any combination thereof is administered.

### (Item 30A)

The method according to any one of the above items, in which the composition completely eliminates the AIDS virus in the subject.

### (Item 31A)

The method according to any one of the above items, in which the composition is not administered before the viral infection.

### (Item 32A)

The method according to any one of the above items, in which the subject is previously inoculated with a BCG vaccine.

### (Item 1B)

A use of a nucleic acid construct containing, in an operable manner, a nucleic acid sequence encoding an antigen protein contained in a virus belonging to the genus Lentivirus or a part thereof and a nucleic acid sequence encoding Ag85B protein in manufacture of a medicament for treating a viral infection in a subject.

### (Item 2B)

The use according to the above item, characterized in that the nucleic acid construct is administered after the viral infection.

### (Item 3B)

The use according to any one of the above items, in which the treatment for the viral infection substantially eliminates the virus from the subject after the viral infection.

### (Item 4B)

The use according to any one of the above items, in which in the treatment for the viral infection, the nucleic acid construct is administered to substantially eliminate the virus from the subject after the viral infection.

### (Item 5B)

The use according to any one of the above items, in which the nucleic acid sequence encoding the antigen protein or part thereof is a nucleic acid sequence encoding an attenuated virus.

### (Item 6B)

The use according to any one of the above items, in which the attenuated virus is an nef-deleted attenuated virus.

### (Item 7B)

The use according to any one of the above items, in which the nucleic acid sequence encoding the Ag85B protein is incorporated into the nucleic acid sequence encoding the attenuated virus.

### (Item 8B)

The use according to any one of the above items, in which the nucleic acid sequence encoding the Ag85B protein is incorporated at a location of a deleted nef gene in a nucleic acid sequence encoding the nef-deleted attenuated virus.

### (Item 9B)

The use according to any one of the above items, in which the virus is an AIDS virus.

### (Item 10B)

The use according to any one of the above items, in which the virus is an attenuated virus of a virus that infects humans.

### (Item 11B)

The use according to any one of the above items, in which the virus is an attenuated virus of an AIDS virus selected from the group consisting of HIV, SIV, SHIV, and FIV.

### (Item 12B)

The use according to any one of the above items, in which the virus is an attenuated HIV.

### (Item 13B)

The use according to any one of the above items, in which the virus is an nef-deleted attenuated HIV.

### (Item 14B)

The use according to any one of the above items, in which the nucleic acid sequence encoding the antigen protein or part thereof includes a nucleic acid sequence at least 90% identical to a nucleic acid sequence containing nucleotides 1 to 9,633 and 10,612 to 11,022 of SEQ ID NO: 3 or a nucleic acid sequence containing nucleotides 1 to 8,786 and 9,765 to 15,182 of SEQ ID NO: 4.

### (Item 15B)

The use according to any one of the above items, in which the nucleic acid construct contains a nucleic acid sequence at least 90% identical to a nucleic acid sequence set forth in SEQ ID NO: 3 or 4.

### (Item 16B)

The use according to any one of the above items, in which the nucleic acid construct contains a nucleic acid sequence set forth in SEQ ID NO: 3 or 4.

### (Item 17B)

The use according to any one of the above items, characterized in that the nucleic acid construct is administered once.

### (Item 18B)

The use according to any one of the above items, characterized in that the nucleic acid construct is administered two or more times.

### (Item 18B-A)

The use according to any one of the above items, characterized in that the nucleic acid construct is administered after administration of an anti-HIV drug.

### (Item 18B-B)

The use according to any one of the above items, in which the anti-HIV drug is selected from the group consisting of a reverse transcriptase inhibitor, a protease inhibitor, an integrase inhibitor, and a CCR5 inhibitor, and preferably from tenofovir, emtricitabine, dolutegravir, zidovudine, lamivudine, sanilvudine, didanosine, abacavir, nevirapine, efavirenz, etravirine, rilpivirine, saquinavir, indinavir, nelfinavir, lopinavir/ritonavir combination, atazanavir, fosamprenavir, darunavir, ritonavir, raltegravir, elvitegravir, maraviroc, or any combination thereof.

### (Item 18B-C)

The use according to any one of the above items, characterized in that the nucleic acid construct is administered after administration of the anti-HIV drug and after discontinuing the administration of the HIV drug.

### (Item 18B-D)

The use according to any one of the above items, characterized in that the nucleic acid construct is administered two or more times after administration of the anti-HIV drug.

### (Item 18B-E)

The use according to any one of the above items, characterized in that the nucleic acid construct is administered three, four, five or more times after administration of the anti-HIV drug.

### (Item 18B-F)

The use according to any one of the above items, characterized in that two or more administrations of the nucleic acid construct are performed at an interval of at least one week or longer.

### (Item 18B-G)

The use according to any one of the above items, characterized in that two or more administrations of the nucleic acid construct are performed at an interval of one week to four weeks.

### (Item 18B-H)

The use according to any one of the above items, characterized in that two or more administrations of the nucleic acid construct are performed at an interval of one to three weeks.

### (Item 18B-I)

The use according to any one of the above items, characterized in that in two or more administrations of the nucleic acid construct, a plasma viral load of the patient is observed and the nucleic acid construct is administered again when a predetermined value is reached.

### (Item 18B-J)

The use according to any one of the above items, characterized in that the nucleic acid construct is administered at a dosage of 5.0 × 10⁵ TCID₅₀ or more or 1.0 × 10⁶ TCID₅₀ or more per administration.

### (Item 18B-K)

The use according to any one of the above items, characterized in that two or more administrations of the nucleic acid construct are performed at 5.0 × 10⁵ TCID₅₀ or more or 1.0 × 10⁶ TCID₅₀ or more per administration at an interval of one week to four weeks.

### (Item 19B)

The use according to any one of the above items, characterized in that the nucleic acid construct is administered once a week, once every two weeks, once every three weeks, once a month, or once every two months.

### (Item 19B-A)

The use according to any one of the above items, characterized in that the nucleic acid construct is administered once a week, once every two weeks, once every three weeks, once a month, once every two months, or at a longer interval.

### (Item 20B)

The use according to any one of the above items, characterized in that the nucleic acid construct is administered at a dose of 1.0 × 10³ TCID₅₀ or more.

### (Item 21B)

The use according to any one of the above items, characterized in that the nucleic acid construct is administered at a dose of 1.0 × 10³ TCID₅₀ or more and less than 5.0 × 10⁴ TCID₅₀.

### (Item 22B)

The use according to any one of the above items, characterized in that the nucleic acid construct is administered once a week at a dose of 1.0 × 10³ TCID₅₀ or more and less than 5.0 × 10⁴ TCID₅₀.

### (Item 23B)

The use according to any one of the above items, characterized in that the nucleic acid construct is administered once at a dose of 1.0 × 10³ TCID₅₀ or more and less than 5.0 × 10⁴ TCID₅₀, and when a virus is detected in a body, the nucleic acid construct is further administered.

### (Item 24B)

The use according to any one of the above items, characterized in that the nucleic acid construct is administered at a dose of 5.0 × 10⁴ TCID₅₀ or more and 5.0 × 10⁶ TCID₅₀ or less.

### (Item 25B)

The use according to any one of the above items, characterized in that the nucleic acid construct is administered once at a dose of 5.0 × 10⁴ TCID₅₀ or more.

### (Item 26B)

The use according to any one of the above items, characterized in that an anti-HIV drug is already administered to the subject.

### (Item 27B)

The use according to any one of the above items, in which an anti-HIV drug is not yet administered to the subject, or the anti-HIV drug is discontinued at the start of administration of the composition.

### (Item 28B)

The use according to any one of the above items, characterized in that as the anti-HIV drug, an anti-HIV drug selected from the group consisting of a reverse transcriptase inhibitor, a protease inhibitor, an integrase inhibitor, and a CCR5 inhibitor is administered.

### (Item 29B)

The use according to any one of the above items, characterized in that as the anti-HIV drug, an anti-HIV drug selected from the group consisting of tenofovir, emtricitabine, dolutegravir, zidovudine, lamivudine, sanilvudine, didanosine, abacavir, nevirapine, efavirenz, etravirine, rilpivirine, saquinavir, indinavir, nelfinavir, lopinavir/ritonavir combination, atazanavir, fosamprenavir, darunavir, ritonavir, raltegravir, elvitegravir, maraviroc, or any combination thereof is administered.

### (Item 30B)

The use according to any one of the above items, in which the composition completely eliminates the AIDS virus in the subject.

### (Item 31B)

The use according to any one of the above items, in which the composition is not administered before the viral infection.

### (Item 32B)

The use according to any one of the above items, in which the subject is previously inoculated with a BCG vaccine.

### (Item 1C)

A nucleic acid construct for treating a viral infection in a subject, the nucleic acid construct containing, in an operable manner, a nucleic acid sequence encoding an antigen protein contained in a virus belonging to the genus Lentivirus or a part thereof and a nucleic acid sequence encoding Ag85B protein.

### (Item 2C)

The nucleic acid construct according to the above item, characterized in that the nucleic acid construct is administered after the viral infection.

### (Item 3C)

The nucleic acid construct according to any one of the above items, in which the treatment for the viral infection substantially eliminates the virus from the subject after the viral infection.

### (Item 4C)

The nucleic acid construct according to any one of the above items, in which in the treatment for the viral infection, the nucleic acid construct is administered to substantially eliminate the virus from the subject after the viral infection.

### (Item 5C)

The nucleic acid construct according to any one of the above items, in which the nucleic acid sequence encoding the antigen protein or part thereof is a nucleic acid sequence encoding an attenuated virus.

### (Item 6C)

The nucleic acid construct according to any one of the above items, in which the attenuated virus is an nef-deleted attenuated virus.

### (Item 7C)

The nucleic acid construct according to any one of the above items, in which the nucleic acid sequence encoding the Ag85B protein is incorporated into the nucleic acid sequence encoding the attenuated virus.

### (Item 8C)

The nucleic acid construct according to any one of the above items, in which the nucleic acid sequence encoding the Ag85B protein is incorporated at a location of a deleted nef gene in a nucleic acid sequence encoding the nef-deleted attenuated virus.

### (Item 9C)

The nucleic acid construct according to any one of the above items, in which the virus is an AIDS virus.

### (Item 10C)

The nucleic acid construct according to any one of the above items, in which the virus is an attenuated virus of a virus that infects humans.

### (Item 11C)

The nucleic acid construct according to any one of the above items, in which the virus is an attenuated virus of an AIDS virus selected from the group consisting of HIV, SIV, SHIV, and FIV.

### (Item 12C)

The nucleic acid construct according to any one of the above items, in which the virus is an attenuated HIV.

### (Item 13C)

The nucleic acid construct according to any one of the above items, in which the virus is an nef-deleted attenuated HIV.

### (Item 14C)

The nucleic acid construct according to any one of the above items, in which the nucleic acid sequence encoding the antigen protein or part thereof includes a nucleic acid sequence at least 90% identical to a nucleic acid sequence containing nucleotides 1 to 9,633 and 10,612 to 11,022 of SEQ ID NO: 3 or a nucleic acid sequence containing nucleotides 1 to 8,786 and 9,765 to 15,182 of SEQ ID NO: 4.

### (Item 15C)

The nucleic acid construct according to any one of the above items, in which the nucleic acid construct contains a nucleic acid sequence at least 90% identical to a nucleic acid sequence set forth in SEQ ID NO: 3 or 4.

### (Item 16C)

The nucleic acid construct according to any one of the above items, in which the nucleic acid construct contains a nucleic acid sequence set forth in SEQ ID NO: 3 or 4.

### (Item 17C)

The nucleic acid construct according to any one of the above items, characterized in that the nucleic acid construct is administered once.

### (Item 18C)

The nucleic acid construct according to any one of the above items, characterized in that the nucleic acid construct is administered two or more times.

### (Item 18C-A)

The nucleic acid construct according to any one of the above items, characterized in that the nucleic acid construct is administered after administration of an anti-HIV drug.

### (Item 18C-B)

The nucleic acid construct according to any one of the above items, in which the anti-HIV drug is selected from the group consisting of a reverse transcriptase inhibitor, a protease inhibitor, an integrase inhibitor, and a CCR5 inhibitor, and preferably from tenofovir, emtricitabine, dolutegravir, zidovudine, lamivudine, sanilvudine, didanosine, abacavir, nevirapine, efavirenz, etravirine, rilpivirine, saquinavir, indinavir, nelfinavir, lopinavir/ritonavir combination, atazanavir, fosamprenavir, darunavir, ritonavir, raltegravir, elvitegravir, maraviroc, or any combination thereof.

### (Item 18C-C)

The nucleic acid construct according to any one of the above items, characterized in that the nucleic acid construct is administered after administration of the anti-HIV drug and after discontinuing the administration of the HIV drug.

### (Item 18C-D)

The nucleic acid construct according to any one of the above items, characterized in that the nucleic acid construct is administered two or more times after administration of the anti-HIV drug.

### (Item 18C-E)

The nucleic acid construct according to any one of the above items, characterized in that the nucleic acid construct is administered three, four, five or more times after administration of the anti-HIV drug.

### (Item 18C-F)

The nucleic acid construct according to any one of the above items, characterized in that two or more administrations of the nucleic acid construct are performed at an interval of at least one week or longer.

### (Item 18C-G)

The nucleic acid construct according to any one of the above items, characterized in that two or more administrations of the nucleic acid construct are performed at an interval of one week to four weeks.

### (Item 18C-H)

The nucleic acid construct according to any one of the above items, characterized in that two or more administrations of the nucleic acid construct are performed at an interval of one to three weeks.

### (Item 18C-I)

The nucleic acid construct according to any one of the above items, characterized in that in two or more administrations of the nucleic acid construct, a plasma viral load of the patient is observed and the nucleic acid construct is administered again when a predetermined value is reached.

### (Item 18C-J)

The nucleic acid construct according to any one of the above items, characterized in that the nucleic acid construct is administered at a dosage of 5.0 × 10⁵ TCID₅₀ or more or 1.0 × 10⁶ TCID₅₀ or more per administration.

### (Item 18C-K)

The nucleic acid construct according to any one of the above items, characterized in that two or more administrations of the nucleic acid construct are performed at 5.0 × 10⁵ TCID₅₀ or more or 1.0 × 10⁶ TCID₅₀ or more per administration at an interval of one week to four weeks.

### (Item 19C)

The nucleic acid construct according to any one of the above items, characterized in that the nucleic acid construct is administered once a week, once every two weeks, once every three weeks, once a month, or once every two months.

### (Item 19C-A)

The nucleic acid construct according to any one of the above items, characterized in that the nucleic acid construct is administered once a week, once every two weeks, once every three weeks, once a month, once every two months, or at a longer interval.

### (Item 20C)

The nucleic acid construct according to any one of the above items, characterized in that the nucleic acid construct is administered at a dose of 1.0 × 10³ TCID₅₀ or more.

### (Item 21C)

The nucleic acid construct according to any one of the above items, characterized in that the nucleic acid construct is administered at a dose of 1.0 × 10³ TCID₅₀ or more and less than 5.0 × 10⁴ TCID₅₀.

### (Item 22C)

The nucleic acid construct according to any one of the above items, characterized in that the nucleic acid construct is administered once a week at a dose of 1.0 × 10³ TCID₅₀ or more and less than 5.0 × 10⁴ TCID₅₀.

### (Item 23C)

The nucleic acid construct according to any one of the above items, characterized in that the nucleic acid construct is administered once at a dose of 1.0 × 10³ TCID₅₀ or more and less than 5.0 × 10⁴ TCID₅₀, and when a virus is detected in a body, the nucleic acid construct is further administered.

### (Item 24C)

The nucleic acid construct according to any one of the above items, characterized in that the nucleic acid construct is administered at a dose of 5.0 × 10⁴ TCID₅₀ or more and 5.0 × 10⁶ TCID₅₀ or less.

### (Item 25C)

The nucleic acid construct according to any one of the above items, characterized in that the nucleic acid construct is administered once at a dose of 5.0 × 10⁴ TCID₅₀ or more.

### (Item 26C)

The nucleic acid construct according to any one of the above items, characterized in that an anti-HIV drug is already administered to the subject.

### (Item 27C)

The nucleic acid construct according to any one of the above items, in which an anti-HIV drug is not yet administered to the subject, or the anti-HIV drug is discontinued at the start of administration of the composition.

### (Item 28C)

The nucleic acid construct according to any one of the above items, characterized in that as the anti-HIV drug, an anti-HIV drug selected from the group consisting of a reverse transcriptase inhibitor, a protease inhibitor, an integrase inhibitor, and a CCR5 inhibitor is administered.

### (Item 29C)

The nucleic acid construct according to any one of the above items, characterized in that as the anti-HIV drug, an anti-HIV drug selected from the group consisting of tenofovir, emtricitabine, dolutegravir, zidovudine, lamivudine, sanilvudine, didanosine, abacavir, nevirapine, efavirenz, etravirine, rilpivirine, saquinavir, indinavir, nelfinavir, lopinavir/ritonavir combination, atazanavir, fosamprenavir, darunavir, ritonavir, raltegravir, elvitegravir, maraviroc, or any combination thereof is administered.

### (Item 30C)

The nucleic acid construct according to any one of the above items, in which the composition completely eliminates the AIDS virus in the subject.

### (Item 31C)

The nucleic acid construct according to any one of the above items, in which the composition is not administered before the viral infection.

### (Item 32C)

The nucleic acid construct according to any one of the above items, in which the subject is previously inoculated with a BCG vaccine.

### (Item 1D)

The composition, the nucleic acid molecule, the method, the use, or the nucleic acid construct according to any one of the above items, in which the composition, the nucleic acid molecule, the method, the use, or the nucleic acid construct is for treating infections with a virus belonging to the genus Lentivirus, and preferably, an AIDS virus, or a viral infection caused by these viruses.

In the present disclosure, it is intended that one or a plurality of the above-mentioned features can be provided in further combination in addition to the specified combination. Still further embodiments and advantages of the present disclosure will be appreciated by those skilled in the art upon reading and understanding the following detailed description, if necessary.

### Brief Description of Drawings

FIG. 1 illustrates an outline of an experiment in Example 3.
FIG. 2 illustrates kinetics of plasma viral loads and CD4⁺ T cell counts in a macaque (#137) to which only an anti-HIV drug is administered.
FIG. 3 illustrates kinetics of plasma viral loads and CD4⁺ T cell counts in a macaque (#139) to which only an anti-HIV drug is administered.
FIG. 4 illustrates kinetics of plasma viral loads and CD4⁺ T cell counts in a macaque (#142) to which a low dose of SHIV-Ag85B is administered two times.
FIG. 5 illustrates kinetics of plasma viral loads and CD4⁺ T cell counts in a macaque (#141) to which a high dose of SHIV-Ag85B is administered once.
FIG. 6 illustrates kinetics of plasma viral loads and CD4⁺ T cell counts in a macaque (#140) to which a high dose of SHIV-Ag85B is administered a plurality of times.
FIG. 7 illustrates kinetics of plasma viral loads and CD4⁺ T cell counts in a macaque (#138) to which each of a low dose and a high dose of SHIV-Ag85B is administered once.
FIG. 8 illustrates an administration schedule in each individual in Example 2.
FIG. 9A illustrates a transition of plasma viral loads in the macaque (#138) to which a low dose of SHIV-Ag85B is administered two times in Example 3.
FIG. 9B illustrates a transition of plasma viral loads in the macaque (#142) to which a high dose of SHIV-Ag85B is administered once in Example 3.
FIG. 10 illustrates that in one macaque (#141) to which a dose of SHIV-Ag85B is administered in Example 3, only a short-term effect is observed in an early stage, and when the SHIV-Ag85B is administered whenever a virus appears in plasma, a long-term inhibitory effect is exhibited from the fifth inoculation.
FIG. 11A is a result of an untreated macaque (#137) to which only an anti-HIV drug is administered in Example 4.
FIG. 11B is a result of an untreated macaque (#139) to which only an anti-HIV drug is administered in Example 4.
FIG. 12 is an experimental protocol for verifying a transition of plasma viral loads when a composition according to the present disclosure is administered after inoculation of an HIV virus in Example 4.
FIG. 13 is a result showing that in Example 4, an anti-HIV drug is effective regardless of individual differences.
FIG. 14 is a diagram showing plasma viral loads of macaques (#164, #165, and #167) to which a high dose (5.0 × 10⁴ TCID₅₀) of SHIV-Ag85B is administered after administration of a high dose of SHIV-Ag85B four times at an interval of one week.
FIG. 15 is a result of plasma viral loads of macaques (#162, #166, and #168) to which a high dose (5.0 × 10⁴ TCID₅₀) of SHIV-NI is administered in Example 5 when a high dose of SHIV-NI is administered four times.
FIG. 16 is a result of plasma viral loads at two weeks after drug cessation in a control group not containing Ag85B in Example 5.

### Mode for Carrying Out the Invention

Hereinafter, the present disclosure will be described. Throughout the present specification, the expression of singular forms is to be understood as including the concept of plural forms unless otherwise stated. Therefore, the singular article (for example, "a", "an", "the", or the like in English) should be understood to include the concept of plural forms unless otherwise stated. In addition, the terms used in the present specification are to be understood as being used in the sense commonly used in the art unless otherwise stated. Therefore, unless defined otherwise, all technical terms and scientific terms used in the present specification have the same meaning as commonly understood by those skilled in the art to which the present disclosure pertains. In the case of conflict, the present specification (including definitions) will control.

### (Definition of terms)

The following lists definitions of terms specifically used in the present specification.

In the present specification, "about" means ±10% of an indicated value.

In the present specification, "Ag85B" refers to an immunogenic protein secreted by an acid-fast bacterium. The acid-fast bacterium exhibits a property that when bacteria are dyed with a pigment, the pigment is not decolorized by an acid, that is, the acid-fast bacterium exhibits resistance to an acid. The acid-fast bacterium (mycobacterium) is broadly classified into Mycobacterium tuberculosis, Mycobacterium leprae, and nontuberculous mycobacteria. A representative source of Ag85B is Mycobacterium Tuberculosis. Ag85B is also referred to as antigen 85B, 85B, Extracellular alpha-antigen, Antigen 85 complex B, Ag85B, Mycolyl transferase 85B, EC 2.3.1.-, Fibronectin-binding protein B, 30 kDa extracellular protein, fbpB, A85B, Major Secretory Protein Antigen 85B, or the like. The Accession number is typically Q847N4. See https://www.uniprot.org/uniprotkb/Q847N4/entry. The ID of the nucleic acid sequence is typically AY207396g, and the ID of the protein sequence is typically AAO62005.1. In the present specification, although shown in SEQ ID NO: 1 (nucleic acid sequence) and SEQ ID NO: 2 (amino acid sequence), it is understood that, without being limited thereto, any immunogenic protein secreted by an acid-fast bacterium is within the scope of the present disclosure.

In the present specification, "operably" refers to a state in which transcription or translation of a nucleic acid sequence is under control of an expression control element, and transcription or translation of the nucleic acid sequence is appropriately controlled so that the nucleic acid sequence is functionally expressed.

In the present specification, "Th1-type immune response" is a cellular immune response mediated by T lymphocytes and refers to a response by cytokines and/or chemokines produced by activated T cells.

In the present specification, "Th2-type immune response" refers to a humoral immune response mediated by secreted antibodies produced in B cells.

In the present specification, "attenuated virus" refers to a virus that has reduced pathogenicity compared to a parent virus, but retains an ability to induce an immune response. Whether or not it is attenuated can be confirmed by toxicity to cells and/or pathogenicity to animals. It is preferably determined by animal experiments or the like. In general, the term "attenuation" is to artificially reduce the toxicity of a pathogen by mutating a gene so that the pathogen loses pathogenicity but retains immunogenicity. In general, attenuation is achieved by attenuating the toxicity of the pathogen through UV radiation, chemical treatment, or continuous high-order subculture in vitro. Genes are artificially altered, for example, by deleting certain nucleotides in known sequences to attenuate toxicity.

In the present specification, "AIDS virus" refers to a virus that causes acquired immunodeficiency syndrome (AIDS), and examples thereof include human immunodeficiency virus (HIV), simian immunodeficiency virus (SIV), simian-human immunodeficiency (chimeric) virus (SHIV), and feline immunodeficiency virus (FIV).

In the present specification, "simian-human immunodeficiency (chimeric) virus (SHIV)" refers to a simian-human immunodeficiency chimeric virus in which at least some of the genes for SIV genes are replaced with those for HIV-1 genes. In the SHIV, in addition to env gene, vpr, rev, tat, and vpu genes can be further replaced with those for HIV-1.

A negative factor (nef) is a protein of about 27 kDa that is encoded on the 3' end of HIV-1 gene. It was originally reported as a factor that suppresses virus proliferation, but thereafter, virus proliferation is rather enhanced in primary cultured cells and in vivo, and is considered to be associated with HIV-1 pathogenicity. HIV-1 Nef protein, anchor domain superfamily (IPR027480), HIV-1 Nef protein, core domain superfamily (IPR027481), and the like are known. Although various vaccine viruses attenuated by genetic disruption of major regulatory genes such as nef, vpx, vpr, and vif have been used, most studies have used the moderately attenuated prototype vaccine strain SIVmac239Δnef. Although nef-deleted attenuated simian immunodeficiency virus (SIV) and simian-human immunodeficiency virus (SHIV) have proven highly effective as vaccines in non-human primate models, these viruses do not have sufficient safety to be used as templates for HIV vaccines in humans. Several studies have demonstrated that insertion of a cytokine or chemokine gene into an attenuated and genetically deleted SIV or SHIV improves immunogenicity and increases viral defenses compared to a safe and less toxic strain, but the nef-deleted attenuated HIV-Ag85B of the present disclosure was unexpectedly found to be able to substantially eliminate the virus, that is, cure the virus to a state in which the virus is completely removed from a living body (typically, a state in which the value is below a limit of detection by a highly sensitive detection method such as PCR, and clinical symptoms are not shown), in a patient who is already infected and has the virus in its body upon administration after infection. In addition, it has been found that an nef-deleted attenuated HIV-Ag85B of the present disclosure or a functional equivalent thereof can be safer, less toxic, and more effective at inducing a Th1-type immune response even in a subject after infection, and can provide a composition of treatment that can withstand practical use against HIV.

The SIV infection model in non-human primates that is also used in the present specification is important for the analysis of the mechanisms of development of acquired immunodeficiency syndrome (AIDS) and for the determination of the efficacy of an HIV vaccine or HIV therapeutic intervention. As the present inventors have also reported, there is pathogenicity of SIVmac and SHIV89.6P in crab-eating macaques in Indonesia, Malaysia, and Philippines. As a result of comparing parameters such as plasma viral loads, peripheral CD4⁺ T cell counts, patterns of viral antigen-specific immune response, and disease outcome, SIV and SHIV have been shown to develop in crab-eating macaques even with different origins. Compared to Indian rhesus macaques, crab-eating macaques from Asia are similarly tolerant to various strains of SIVmac and SHIV and have longer survival after infection with SIV or SHIV, indicating that the crab-eating macaques are a model that closely resembles the progression of HIV-1 disease in humans.

In the present specification, "completely eliminating a virus" refers to a state in which a virus is completely removed from a living body. The state in which the virus is completely removed from the living body means that the virus is below a limit of detection by a highly sensitive detection method such as PCR and no clinical symptoms are observed.

In the present specification, "vaccine" means a substance that contains or encodes an antigen that provides active immunity to a substance containing an antigen but does not cause disease. In the present specification, a vaccine refers to one that is used prophylactically. In the present specification, "DNA vaccine" means a nucleic acid encoding a vaccine antigen, and such a generic name is used since DNA (in particular, plasmid DNA) is mainly used. Note that, as an embodiment using a nucleic acid, there is also an aspect in which the nucleic acid is incorporated into a viral vector or the like and delivered into a living body, and in this case, it is understood that the nucleic acid can be provided in a form of a nucleic acid other than DNA. Such a case is also referred to as "nucleic acid vaccine". Usually, the DNA vaccine takes the form of plasmid DNA, but when the DNA vaccine is subcutaneously administered in the form of plasmid DNA, the plasmid DNA is incorporated into subcutaneous cells, and a target antigen protein is produced in the cells.

In the present specification, "antigen" (also referred to as Ag) refers to any substrate that can be specifically bound by an antibody molecule. In the present specification, "immunogen" refers to an antigen that can initiate lymphocyte activation that produces an antigen-specific immune response.

In the present specification, "remission" refers to a healthy state in which a virus is completely eliminated and does not require administration of a therapeutic drug.

In the present specification, "treatment" of "viral infection" refers to reducing at least one symptom due to the viral infection, slowing progression of at least one symptom, or ameliorating at least one symptom.

In the present specification, "substantially eliminating a virus" refers to clinically determining that a virus cannot be detected and is not developed.

In the present specification, "subject" refers to a mammal, including a human patient, which is the subject of the treatment of the present disclosure.

In the present specification, "nucleic acid construct" refers to a DNA or RNA molecule containing a nucleotide sequence encoding a protein.

In the present specification, "deletion" refers to disappearance of a gene. The deletion also includes that a part of a gene disappears and does not function.

In the present specification, "X time-administration" refers to × time-administration during an observation period.

In the present specification, "TCID₅₀" refers to a 50% tissue culture infectious dose, and means an amount of virus infecting 50% of cells.

In the present specification, "anti-HIV drug is already administered" refers to that the anti-HIV drug is administered before the nucleic acid construct of the present disclosure is administered.

In the present specification, "anti-HIV drug is not yet administered" refers to that the anti-HIV drug is not administered when the nucleic acid construct of the present disclosure is administered.

In the present specification, "reverse transcriptase inhibitor" refers to a drug that blocks an enzymatic function of reverse transcriptase, prevents synthesis of double-stranded viral DNA, and inhibits proliferation of HIV.

In the present specification, "protease inhibitor" refers to a drug that binds to an enzyme active site of a protease and loses its activity. A functional protein of HIV is first produced as a complex protein and functions only when it is cleaved at a specific site by the protease of HIV itself. The activity of the protease is eliminated by the protease inhibitor, and as a result, the virus cannot be completed and loses infectivity.

In the present specification, "integrase inhibitor" refers to a drug having an action of inhibiting integrase, thereby inhibiting a reaction in which HIV incorporates its own gene into a host chromosome, and causing an infection to fail. As a result, infectious virus particles are not newly produced, and thus infection spread is prevented.

In the present specification, "CCR5 inhibitor" refers to a drug that inhibits a C-C chemokine receptor 5 (CCR5), which is a co-receptor utilized when HIV enters cells.

In the present specification, "completely eliminating an AIDS virus" refers to that it is determined that a virus such as an AIDS virus cannot be detected by any detection method in addition to a clinical method.

In the present specification, "nucleic acid construct", "construct", and "gene construct" are used interchangeably and are nucleic acid molecules containing a plurality of sets of aligned nucleic acids isolated from a naturally occurring gene or combined in a form that does not naturally present.

Amino acids can be mentioned in the present specification by either their commonly known three letter symbols or their one character symbols recommended by the IUPAC-IUB Biochemical Nomenclature Commission. Similarly, nucleotides can be mentioned by their commonly recognized one character codes. In the present specification, a comparison of similarity, identity, and homology of an amino acid sequence and a base sequence is calculated by using a sequence analysis tool BLAST and default parameters. A search for identity can be performed, for example, using BLAST2.2.28 (published on April 2, 2013) (Proc. Natl. Acad. Sci. USA 90: 5873-5877, 1993) of the NCBI. In the present specification, a value of identity generally refers to a value obtained when aligned under the default conditions using BLAST described above. However, when a higher value is obtained by changing a parameter, the highest value is set as the value of identity. In a case where identity is evaluated in a plurality of regions, the highest value in the plurality of regions is set as the value of identity. Similarity is a numerical value calculated by taking into consideration a similar amino acid in addition to identity. Blastp can be used as a default setting for algorithms when comparing amino acid sequences in BLAST. The measurement results are quantified as Positives or Identities. The homology of amino acid sequences or base sequences can be determined by the algorithm BLAST by Karlin and Altschul. Based on this algorithm, programs called BLASTN and BLASTX have been developed (Altschul et al. J. Mol. Biol. 215: 403-410, 1990). In a case where the base sequence is analyzed by BLASTN based on BLAST, the parameters are set to, for example, score = 100 and wordlength = 12. In addition, in a case where the amino acid sequence is analyzed by BLASTX based on BLAST, the parameters are set to, for example, score = 50 and wordlength = 3. In a case where BLAST and Gapped BLAST programs are used, default parameters of each program are used. Specific methods of these analysis methods are known (http://www.ncbi.nlm.nih.gov.).

Nucleic acids or proteins used in the present disclosure can contain sequences in which one or a plurality of amino acids or nucleotides are substituted, deleted, and/or added in a target amino acid or base sequence. Here, "one or a plurality of" in a full-length amino acid sequence of a chimeric protein is usually within 50 amino acids, preferably within 30 amino acids, and more preferably within 10 amino acids (for example, within 5 amino acids, within 3 amino acids, or one amino acid). In addition, "one or a plurality of" in an amino acid sequence of a domain is usually within 6 amino acids, preferably within 5 amino acids, and more preferably within 4 amino acids (for example, within 3 amino acids, within 2 amino acids, or one amino acid). In a case where the biological activity of the present disclosure of the chimeric protein is maintained, it is desirable that a mutated amino acid residue is mutated to another amino acid in which the properties of the amino acid side chain are conserved. Examples of the properties of the amino acid side chain include a hydrophobic amino acid (A, I, L, M, F, P, W, Y, or V), a hydrophilic amino acid (R, D, N, C, E, Q, G, H, K, S, or T), an amino acid having an aliphatic side chain (G, A, V, L, I, or P), an amino acid having a hydroxyl group-containing side chain (S, T, or Y), an amino acid having a sulfur atom-containing side chain (C or M), an amino acid having a carboxylic acid-amide-containing side chain (D, N, E, or Q), an amino acid having a base-containing side chain (R, K, or H), and an amino acid having an aromatic group-containing side chain (H, F, Y, or W) (the parentheses each represent a single letter of the amino acid). These properties are also referred to in the present specification as "conservative substitutions". Note that it is known that a protein containing an amino acid sequence modified by deletion, addition, and/or substitution of one or a plurality of amino acid residues with respect to a certain amino acid sequence maintains its biological activity (Mark, D. F. et al., Proc. Natl. Acad. Sci. USA (1984) 81, 5662-5666, Zoller, M. J. & Smith, M. Nucleic Acids Research (1982) 10, 6487-6500, Wang, A. et al., Science 224, 1431-1433, Dalbadie-McFarland, G. et al., Proc. Natl. Acad. Sci. USA (1982) 79, 6409-6413). Therefore, in an embodiment of the present disclosure, "several" may be, for example, 10, 8, 6, 5, 4, 3, or 2, or may be equal to or less than any of these values. A chimeric protein with deletion or the like can be produced, for example, by a site-directed mutagenesis method, a random mutagenesis method, biopanning using an antibody phage library, or the like. In the present disclosure, "70% or more" may be, for example, 70, 75, 80, 85, 90, 95, 96, 97, 98, 99% or more, and the like, "80% or more" may be, for example, 80, 85, 90, 95, 96, 97, 98, or 99% or more, "90% or more" may be, for example, 90, 95, 96, 97, 98, or 99% or more, and these values may be within a range of any two of these values. "Homology" may be calculated by a percentage of amino acids that are homologous in two or a plurality of amino acid sequences according to methods known in the art. Before calculating the percentage, the amino acid sequences of the amino acid sequence group to be compared are aligned, and if necessary, a gap is introduced into a part of the amino acid sequence to maximize the percentage of identical amino acids. Methods for alignment, percentage calculation methods, comparison methods, and computer programs related thereto are conventionally well known in the art (for example, BLAST, GENETYX, and the like). In the case of "identity", a percentage of identical amino acids is calculated, and in the case of "similarity", a percentage of similar amino acids is calculated. Examples of the similar amino acids include, but are not limited to, amino acids capable of conservative substitutions.

In the present disclosure, a part of the construct specifically described in the present disclosure is also encompassed within the scope of the present disclosure. In the present specification, "part" or "fragment" refers to a polypeptide or polynucleotide having a sequence length of 1 to n-1 with respect to the full length polypeptide or polynucleotide (having a length n). The length of the fragment can be appropriately changed in accordance with the object, examples of a lower limit of such a length include 3, 4, 5, 6, 7, 8, 9, 10, 15, 20, 25, 30, 40, 50, and more amino acids for a polypeptide, and a length represented by an integer that is not specifically listed herein (for example, 11 or the like) can also be suitable as the lower limit. In addition, examples of the length include 5, 6, 7, 8, 9, 10, 15, 20, 25, 30, 40, 50, 75, 100, and more nucleotides for a polynucleotide, and a length represented by an integer that is not specifically listed herein (for example, 11 or the like) can also be suitable as the lower limit. In the present specification, it is understood that such a fragment is within the scope of the present disclosure, for example, in a case where a full length one functions as a vaccine, as long as the fragment itself also functions as a vaccine. In the present disclosure, "functional equivalent" of one specifically described in the present disclosure (such as a construct) is also encompassed within the scope of the present disclosure. In the present specification, the "functional equivalent" refers to any entity having the same target function as a target entity but differs in structure from the target entity.

**In** accordance with the present disclosure, in the present specification, the term "activity" refers to a function of a molecule in the broadest sense. Activity, although not intended to be limited, generally includes a biological function, biochemical function, physical function, and chemical function of a molecule. Examples of the activity include enzymatic activity, an ability to interact with another molecule, an ability to activate, promote, stabilize, inhibit, suppress, or destabilize a function of another molecule, stability, and an ability to localize at a specific position in a cell. When applicable, the term also relates to a function of a protein complex in the broadest sense. **In** the present specification, "biological activity" includes activation of a photoreaction and the like.

In the present specification, the "functional equivalent" refers to any entity having the same target function as a target entity but differs in structure from the target entity. Therefore, it is understood that a "protein encoded by HIV virus" or a functional equivalent of a chimera thereof is not the protein encoded by the HIV virus or the chimera thereof itself, but encompasses a mutant or variant of the protein encoded by the HIV virus or the chimera thereof (for example, an amino acid sequence variant or the like) having the biological effect of the protein encoded by the HIV virus or the chimera thereof, and a mutant or variant of the protein encoded by the HIV virus or the antibody thereof itself or the protein encoded by the HIV virus or the chimera thereof at the time of action (for example, a nucleic acid encoding a protein encoded by an HIV virus or a chimera thereof or a protein encoded by an HIV virus or a chimeric mutant or variant thereof, and a vector, a cell, or the like containing the nucleic acid). As the functional equivalent of the present disclosure, an amino acid sequence in which one or a plurality of amino acids are inserted, substituted, and/or deleted, or added to one or both terminals thereof can be used. In the present specification, "the amino acid sequence in which one or a plurality of amino acids are inserted, substituted, and/or deleted, or added to one or both terminals thereof" refers to an alteration with a substitution of a plurality of amino acids or the like to the extent that can occur naturally by a well-known technical method such as site-directed mutagenesis or natural mutation. A modified amino acid sequence can be, for example, an amino acid sequence in which 1 to 30, preferably 1 to 20, more preferably 1 to 9, further preferably 1 to 5, and particularly preferably 1 or 2 amino acids are inserted, substituted, deleted, or added to one or both terminals thereof. The modified amino acid sequence may preferably be an amino acid sequence containing one or a plurality of (preferably 1 or several, or 1, 2, 3, or 4) conservative substitutions in the amino acid sequence of the protein encoded by the HIV virus.

In the present specification, "treatment" refers to preventing, preferably maintaining of the current condition, more preferably alleviating, and still more preferably disappearing deterioration of a disease or disorder (for example, due to a viral infection) when the disease or disorder is in such a state, and includes exerting a symptom ameliorating effect on a disease of a patient or one or more symptoms associated with the disease. Performing diagnosis in advance and then performing an appropriate treatment is referred to as a "companion treatment", and a diagnostic drug therefor may be referred to as a "companion diagnostic drug".

In the present specification, "therapeutic (treatment) drug (agent)" refers in a broad sense to any drug capable of treating a target condition (for example, a retinal degeneration disease or the like). In an embodiment of the present disclosure, "therapeutic drug" may be a pharmaceutical composition containing an active ingredient and one or more pharmacologically acceptable carriers. The pharmaceutical composition can be prepared, for example, by any method known in the technical field of formulation by mixing the active ingredient and the carrier. In addition, the therapeutic drug is not limited in the form of use as long as it is used for treatment, and may be an active ingredient alone or a mixture of an active ingredient and an arbitrary ingredient. In addition, the shape of the carrier is not particularly limited, and may be, for example, a solid or a liquid (for example, a buffer).

The present disclosure can be provided as a kit. In the present specification, "kit" refers to a unit that is usually divided into two or more compartments and is provided with portions to be provided (for example, a plurality of nucleic acid constructs, lyophilized drugs and buffers for administration, manual, and the like). Such a kit preferably includes an instruction or manual describing how to use the provided portions (for example, how to use a nucleic acid construct or how a reagent should be handled). The present specification also includes an instruction and the like describing how to use.

The "active ingredient" in the present specification refers to an ingredient contained in an amount necessary for obtaining an effect such as treatment or progress suppression for which the composition of the present disclosure or the like is intended, and other ingredients may also be contained as long as the effect is not impaired to a level below a desired level. In addition, the medicament, composition, and the like of the present disclosure may be formulated. In addition, an administration route of the medicament, composition, and the like of the present disclosure may be either oral or parenteral administration, and can be appropriately set according to the form of the formulation and the like. The construct of the present disclosure can be used as an active ingredient.

In the present specification, the "instruction" (including package inserts, labels utilized by the U. S. FDA, and the like) is a document with an explanation of the method of use of the present disclosure for a physician or other users. The instruction has a description instructing administration of the medicament and the like of the present disclosure. In addition, the instruction may have a description instructing intravenous administration and the like (for example, by injection and the like) as an administration site. The instruction is prepared in accordance with a format defined by the regulatory agency of the country in which the present disclosure is practiced (for example, the Ministry of Health, Labor and Welfare in Japan, Food and Drug Administration (FDA) in the United States or the like), with an explicit description showing approval by the regulatory agency. The instruction is a so-called package insert or label and is usually provided in, but is not limited to, a paper medium, and for example, the instruction can also be provided in a form such as an electronic medium (for example, a web site, PDF, or an e-mail provided on the Internet).

### (Preferred embodiments)

Although preferred embodiments are described below, it is to be understood that these embodiments are illustrative of the present disclosure and that the scope of the present disclosure is not limited to these preferred embodiments. It should be understood that those skilled in the art can also easily implement modifications, changes, and the like within the scope of the present disclosure with reference to the following preferred examples. For these embodiments, those skilled in the art can appropriately combine any embodiments.

In one aspect, the present disclosure provides a composition for treating a viral infection in a subject, and a method, use, and the like related thereto, the composition containing a nucleic acid construct containing, in an operable manner, a nucleic acid sequence encoding an antigen protein contained in a virus belonging to the genus Lentivirus or a part thereof and a nucleic acid sequence encoding Ag85B protein.

In one preferred embodiment, the subject in the present disclosure is a subject after a viral infection, and the composition of the present disclosure is administered after an infection.

In a preferred embodiment, in the treatment for the viral infection of the present disclosure, the composition is administered to substantially eliminate, and preferably to completely eliminate the virus from the subject after the viral infection.

Here, "substantially" "eliminating" a virus means that it is clinically determined that a virus such as an AIDS virus cannot be detected and is not developed.

Here, "completely" "eliminating" a virus means that it is determined that a virus such as an AIDS virus cannot be detected by any detection method (for example, by a highly sensitive PCR test) and clinical symptoms are not exhibited.

Thus, in a preferred embodiment, the present disclosure provides a composition that is administered from a virally infected subject for the purpose of substantially eliminating the virus.

In one embodiment, the nucleic acid sequence encoding the antigen protein or part thereof used is a nucleic acid sequence encoding an attenuated virus, and preferably, the attenuated virus is an nef-deleted attenuated virus. Since the nef gene is a gene responsible for immune evasion of the AIDS virus, deletion of this part is attenuated by the immune function of the living body. Conventionally, an attenuated virus is not expected to be used with such an adjuvant antigen.

In one embodiment, the nucleic acid sequence encoding the Ag85B protein used in the present disclosure is incorporated into the nucleic acid sequence encoding the attenuated virus of the present disclosure. Since the nef gene is a gene responsible for immune evasion of the AIDS virus, deletion of this part is attenuated by the immune function of the living body. The addition of Ag85B induces strong cellular immunity, induces a strong immune reaction, and attenuates the virus more.

In one embodiment, the nucleic acid sequence encoding the Ag85B protein used in the present disclosure is incorporated at a location of a deleted nef gene in a nucleic acid sequence encoding the nef-deleted attenuated virus. Since the nef gene is a gene responsible for immune evasion of the AIDS virus, deletion of this part is attenuated by the immune function of the living body. The addition of Ag85B induces strong cellular immunity, induces a strong immune reaction, and attenuates the virus more.

In one embodiment, the virus used is an AIDS virus.

In one embodiment, the virus used is an attenuated virus of a virus that infects humans.

In one embodiment, the virus used is an attenuated virus of an AIDS virus selected from the group consisting of human immunodeficiency virus (HIV), simian immunodeficiency virus (SIV), simian-human immunodeficiency chimeric virus (SHIV), and feline immunodeficiency virus (FIV).

In a preferred embodiment, the virus is an HIV virus.

Human immunodeficiency virus (HIV) is genetically broadly classified into two types: Type I HIV (HIV-1) and Type II HIV (HIV-2). HIV-1 is the most prevalent type in the world including Japan, and is classified into three types: Group M (Main), which accounts for the majority of cases, and Groups O (Outlier) and N (New) recognized in localized areas, and Group M is further classified into nine subtypes (A to D, F to H, J, and K). On the other hand, HIV-2 is classified into subtypes A to G, and exists mainly only in West Africa, but infection cases have also been reported in France, the United States, the West India region, and South Korea.

In a certain embodiment, the virus is a strain of HIV-1, and examples thereof include, but are not limited to, BaL strain, IIIB strain, RF strain, GB8 strain, U455 strain, ROD strain, and mutant strains thereof.

In a preferred embodiment, the virus can be an attenuated HIV. In a more preferred embodiment, the virus can be an nef-deleted attenuated HIV.

In one embodiment, the virus used is an nef-deleted attenuated SHIV. Since the nef gene is a gene responsible for immune evasion of the AIDS virus, deletion of this part is attenuated by the immune function of the living body. The addition of Ag85B induces strong cellular immunity, induces a strong immune reaction, and attenuates the virus more.

The nucleic acid sequence encoding the antigen protein or part thereof used is a nucleic acid sequence at least 90% identical, at least 95% identical, at least 96% identical, at least 97% identical, at least 98% identical, or at least 99% identical to a nucleic acid sequence containing nucleotides 1 to 9,633 and 10,612 to 11,022 of SEQ ID NO: 3, or a nucleic acid sequence containing nucleotides 1 to 8,786 and 9,765 to 15,182 of SEQ ID NO: 4.

In a preferred embodiment, the nucleic acid construct used is a nucleic acid sequence at least 90% identical, at least 95% identical, at least 96% identical, at least 97% identical, at least 98% identical, or at least 99% identical to a nucleic acid sequence set forth in SEQ ID NO: 3 or 4, and preferably, the nucleic acid construct used contains the nucleic acid sequence set forth in SEQ ID NO: 3 or 4. Further advantageously, the nucleic acid construct used contains one or several modifications in the nucleic acid sequence set forth in SEQ ID NO: 3 or 4 (in particular, the modification can be a conservative substitution, and a modification site can be a site that does not affect the activity), and particularly advantageously is the nucleic acid sequence set forth in SEQ ID NO: 3 or 4. In particular, it has been demonstrated for the first time that the construct containing the sequence of SEQ ID NO: 3 or 4 of the present disclosure can provide a composition of treatment that is safer and less toxic and can withstand practical use against HIV, and thus clinical significance is high.

"One or several" in the amino acid sequence encoded by the construct of the present disclosure is usually within 6 amino acids, preferably within 5 amino acids, and more preferably within 4 amino acids (for example, within 3 amino acids, within 2 amino acids, or one amino acid). In a case where the biological activity of the present disclosure of the chimeric protein is maintained, it is desirable that a mutated amino acid residue is mutated to another amino acid in which the properties of the amino acid side chain are conserved. When modified, conservative substitutions are preferred in terms of the properties of the amino acid side chain. In an embodiment of the present disclosure, "several" may be, for example, 10, 8, 6, 5, 4, 3, or 2, or may be equal to or less than any of these values. A chimeric protein with deletion or the like can be produced, for example, by a site-directed mutagenesis method, a random mutagenesis method, biopanning using an antibody phage library, or the like. As the site-directed mutagenesis method, for example, KOD-Plus-Mutagenesis Kit (TOYOBO CO., LTD.) can be used.

### (Dosage and administration)

In various embodiments, the composition of the present disclosure can be used in a variety of dosage and administration.

In one embodiment, for the composition of the present disclosure, the nucleic acid construct is administered once or two or more times, for example, three, four, five or more times.

Although single administration is preferred in terms of convenience, repeated administration, high dose administration, or a combination thereof may be used to achieve a reliable therapeutic effect. Those skilled in the art can appropriately determine the number of administrations, frequency, and dosage.

For example, in the present disclosure, the nucleic acid construct or the composition is administered once a week, once every two weeks, once every three weeks, once a month, or once every two months, once every three months, once every four months, once every six months, or once a year.

In one embodiment, the composition or the nucleic acid construct of the present disclosure is administered at a dose of 1.0 × 10³ TCID₅₀ or more. Alternatively, in another embodiment, the composition or the nucleic acid construct of the present disclosure is administered at 1.0 × 10¹ TCID₅₀ or more, 2.0 × 10¹TCID₅₀ or more, 3.0 × 10¹ TCID₅₀ or more, 4.0 × 10¹TCID₅₀ or more, 5.0 × 10¹ TCID₅₀ or more, 6.0 × 10¹ TCID₅₀ or more, 7.0 × 10¹ TCID₅₀ or more, 8.0 × 10¹ TCID₅₀ or more, 9.0 × 10¹ TCID₅₀ or more, 1.0 × 10² TCID₅₀ or more, 2.0 × 10² TCID₅₀ or more, 3.0 × 10² TCID₅₀ or more, 4.0 × 10² TCID₅₀ or more, 5.0 × 10² TCID₅₀ or more, 6.0 × 10² TCID₅₀ or more, 7.0 × 10² TCID₅₀ or more, 8.0 × 10² TCID₅₀ or more, 9.0 × 10² TCID₅₀ or more, 1.0 × 10³ TCID₅₀ or more, 2.0 × 10³ TCID₅₀ or more, 3.0 × 10³ TCID₅₀ or more, 4.0 × 10³ TCID₅₀ or more, 5.0 × 10³ TCID₅₀ or more, 6.0 × 10³ TCID₅₀ or more, 7.0 × 10³ TCID₅₀ or more, 8.0 × 10³ TCID₅₀ or more, 9.0 × 10³ TCID₅₀ or more, 1.0 × 10⁴ TCID₅₀ or more, 2.0 × 10⁴ TCID₅₀ or more, 3.0 × 10⁴ TCID₅₀ or more, 4.0 × 10⁴ TCID₅₀ or more, 5.0 × 10⁴ TCID₅₀ or more, 6.0 × 10⁴ TCID₅₀ or more, 7.0 × 10⁴ TCID₅₀ or more, 8.0 × 10⁴ TCID₅₀ or more, 9.0 × 10⁴ TCID₅₀ or more, or 1.0 × 10⁵ TCID₅₀ or more.

In one embodiment, the nucleic acid construct or the composition is administered at a dose of 1.0 × 10³ TCID₅₀ or more and less than 5.0 × 10⁴ TCID₅₀.

In some embodiments, the low dose, high dose, or both may be administered two or more times per week, for example, two, three, four, five or more times. In a certain embodiment, a low dose may be administered, and then, a high dose may be administered. In a certain embodiment, a high dose may be administered a plurality of times, for example, four times.

In one embodiment, the nucleic acid construct or the composition is administered once at a dose of 1.0 × 10³ TCID₅₀ or more and less than 5.0 × 10⁴ TCID₅₀, and when a virus is detected in a body, the nucleic acid construct is further administered. Detection of the virus can be performed by an antigen test, a PCR test, or the like.

In one embodiment, the nucleic acid construct or the composition is administered, as a low dose, at a dose of 5.0 × 10⁴ TCID₅₀ or more and 5.0 × 10⁶ TCID₅₀ or less.

In one embodiment, the nucleic acid construct or the composition is administered once, as a high dose, at a dose of 5.0 × 10⁴ TCID₅₀ or more.

In one embodiment, the nucleic acid construct or the composition can be administered to a subject to which an anti-HIV drug is already administered. Examples of the already administered anti-HIV drug include a reverse transcriptase inhibitor, a protease inhibitor, an integrase inhibitor, and a CCR5 inhibitor, and for example, tenofovir, emtricitabine, dolutegravir, zidovudine, lamivudine, sanilvudine, didanosine, abacavir, nevirapine, efavirenz, etravirine, rilpivirine, saquinavir, indinavir, nelfinavir, lopinavir/ritonavir combination, atazanavir, fosamprenavir, darunavir, ritonavir, raltegravir, elvitegravir, maraviroc, or any combination thereof.

In one embodiment, the nucleic acid construct or the composition can be administered to a subject to which an anti-HIV drug is not yet administered or a subject for which an anti-HIV drug is discontinued at the start of administration of the composition.

In one embodiment, the other anti-HIV drug used is selected from the group consisting of a reverse transcriptase inhibitor, a protease inhibitor, an integrase inhibitor, and a CCR5 inhibitor.

In one embodiment, the anti-HIV drug that can be used with the nucleic acid construct or the composition of the present disclosure can be an anti-HIV drug selected from the group consisting of tenofovir, emtricitabine, dolutegravir, zidovudine, lamivudine, sanilvudine, didanosine, abacavir, nevirapine, efavirenz, etravirine, rilpivirine, saquinavir, indinavir, nelfinavir, lopinavir/ritonavir combination, atazanavir, fosamprenavir, darunavir, ritonavir, raltegravir, elvitegravir, maraviroc, or any combination thereof.

In one embodiment, the nucleic acid construct or the composition completely eliminates the AIDS virus in the subject, which was not conventionally provided. In addition, even in an individual in which the virus cannot be completely eliminated, continuous administration of the nucleic acid construct or the composition of the present disclosure can continuously suppress onset and can maintain health.

In one embodiment, the nucleic acid construct or the composition is not administered to the subject before a viral infection. It has not been previously known that a target infectious virus such as an AIDS virus is completely eliminated in such a subject.

### (Method for producing construct)

The construct of the present disclosure can be produced as follows. Representative examples thereof are described in the Examples.

Viruses are isolated from patients and viruses are produced from molecules other than nef (for example, gag, pol, vif, vpx, vpr, vpu, tat, env, rev, or a combination thereof). At that time, the Ag85B gene is inserted into the nef region. Ag85B can be genetically separated from a BCG vaccine. Insertion is made into the nucleic acid sequence of the virus by an integration plasmid.

A construct may be produced by incorporating Ag85B into a known viral strain. For example, for Type I HIV (HIV-1) virus, typically, HIV-1 NL432 strain can be used, but other examples include, but are not limited to, BaL strain, IIIB strain, RF strain, GB8 strain, U455 strain, ROD strain, and mutant strains thereof.

### (Use of construct)

The construct of the present disclosure can be used for treating HIV. In one aspect, the present disclosure provides a composition for treating a viral infection in a subject, the composition containing a nucleic acid construct containing, in an operable manner, a nucleic acid sequence encoding an antigen protein contained in a virus belonging to the genus Lentivirus or a part thereof and a nucleic acid sequence encoding Ag85B protein. The nucleic acid construct of the present disclosure is capable of treating a viral infection without relying on a Th2-type immune response.

In some embodiments, the nucleic acid sequence encoding the antigen protein or part thereof can include a nucleic acid sequence at least 80%, 85%, 90%, 95%, 96%, 97%, 98%, 99%, or 100% identical to a nucleic acid sequence containing nucleotides 1 to 9,633 and 10,612 to 11,022 of SEQ ID NO: 3, or a nucleic acid sequence containing nucleotides 1 to 8,786 and 9,765 to 15,182 of SEQ ID NO: 4.

### (Pharmaceutical use)

In one aspect, the present disclosure provides a pharmaceutical composition for treating a viral infection in a subject, the pharmaceutical composition containing a nucleic acid construct containing, in an operable manner, a nucleic acid sequence encoding an antigen protein contained in the virus belonging to the genus Lentivirus or a part thereof and a nucleic acid sequence encoding Ag85B protein. The treatment for the viral infection can be achieved by inducing a Th1-type immune response without relying on a Th2-type immune response. Treatment after an HIV infection was achieved for the first time by the present invention.

The composition of the present disclosure is capable of completely eliminating a virus in a subject. In particular, in the HIV infection, the complete elimination of the virus from a subject has not been achieved so far. The complete elimination of the virus is achieved by a Th1-type immune response.

In a preferred embodiment, the subject is previously inoculated with a BCG vaccine. Since inoculation of a BCG vaccine is expected to enhance the adjuvant effect of Ag85B, the therapeutic effect on HIV by HIV-Ag85B is expected to be high in a BCG inoculation group. The BCG vaccine is not necessarily required in some countries (such as the United States) because of contraindications or the like. A sufficient effect is also expected in a BCG non-inoculation group.

### (Medicament and method of treatment)

In one aspect, the present disclosure provides a method of treatment by administering the composition or the medicament of the present disclosure. In one embodiment, the composition or the nucleic acid construct of the present disclosure is administered by injection. In another embodiment, the composition or the nucleic acid construct of the present disclosure is administered into a body. In a certain embodiment, the composition or the nucleic acid construct of the present disclosure can be provided with a preservation solution. In some embodiments, the preservation solution can be a buffer. In other embodiments, the composition or the nucleic acid construct of the present disclosure can be provided in a container. In a certain embodiment, the container containing the composition or the nucleic acid construct of the present disclosure can be a syringe.

Administration of a drug associated with the attenuated virus of the present disclosure can be performed by any suitable means that, in combination with other ingredients, results in a concentration of a therapeutic drug effective in ameliorating or alleviating HIV. The drug may be contained in any suitable carrier substance in any suitable amount, and is generally present in an amount of 1 to 95 wt% of the total weight of the composition. The composition may be provided in a dosage form suitable for a parenteral (for example, subcutaneous, intravenous, intramuscular, or intraperitoneal) administration route. The pharmaceutical composition can be formulated according to the conventional pharmaceutical practice (see, for example, Remington: The Science and Practice of Pharmacy (20th ed.), ed. A. R. Gennaro, Lippincott Williams & Wilkins, 2000 and Encyclopedia of Pharmaceutical Technology, eds. J. Swarbrick and J. C. Boylan, 1988-1999, Marcel Dekker, New York). The pharmaceutical composition of the present disclosure can be formulated to release an active compound approximately immediately after administration, or at a predetermined time or a predetermined period after administration.

When the present disclosure is used for parenteral administration, the pharmaceutical composition may be formulated in a state of being contained in a unit dose ampule or a multi-dose container or tube, and may also contain additives such as a stabilizer, a buffering agent, a preservative, and a tonicity agent. In addition, the formulation in the case of parenteral administration may be formulated into a powder that can be redissolved with an appropriate carrier (sterile water or the like) at the time of use. Examples of the parenteral administration include intravenous administration, intramuscular administration, and subcutaneous administration, and intravenous administration is preferable. The construct and active ingredient described in the present specification can be administered together with a carrier. The carrier includes a diluent, an adjuvant, an excipient, or a medium. The composition can also contain a small amount of a wetting agent, an emulsifying agent, or a pH buffering agent, if necessary. These compositions can take the form of a solution, a suspension, an emulsion, a tablet, a pill, a capsule, a powder, a sustained release formulation, a combination thereof, and the like.

A pharmaceutically acceptable carrier that can be used in the present disclosure refers to a medium containing the construct or drug described in the present specification that can be injected into a subject without any side effects. The pharmaceutically acceptable carrier includes a sterile liquid, for example, water and oil, and oil includes a petroleum-derived oil, an animal-derived oil, a plant-derived oil, or a synthetically-derived oil, for example, peanut oil, soybean oil, mineral oil, sesame oil, a combination thereof, or the like. A preferred pharmaceutically acceptable carrier includes starch, glucose, lactose, sucrose, gelatin, malt, rice, flour, chalk, silica gel, sodium stearate, glycerol monostearate, talc, sodium chloride, dry skimmed milk, glycerol, propylene, glycol, water, ethanol, a combination thereof, and the like. Other examples of the preferred pharmaceutical carrier are described in "Remington's Pharmaceutical Sciences" by E. W. Martin. Administration of the composition of the present disclosure can be by any route typically used for inoculation of the composition of the present disclosure, including a topical route, a subcutaneous route, an intravenous route, an intramuscular route, an intradermal route, an intraperitoneal route, an oral route, an inhalation route, or a combination thereof.

The composition described in the present specification can be formulated into a nucleic acid medicament. The nucleic acid medicament described in the present specification includes a vector described in the present specification containing DNA encoding fully long genomic RNA molecules of an infectious non-pathogenic and/or attenuated virus operably connected to a promoter appropriate for expression in eukaryotic cells.

In one certain embodiment, the construct, the medicament, and the like of the present disclosure are administered once or two or more times during a treatment period. As described in the examples, it is confirmed that the medicament and the like of the present disclosure exhibits its effect by being administered at least once, and it is considered that compliance to the patient is also excellent.

### (General technique)

The molecular biological technique, the biochemical technique, and the microbiological technique used in the present specification are well known and commonly used in the art, and are described, for example, in Current Protocols in Molecular Biology (http://onlinelibrary.wiley.com/book/10.1002/0471142727) and Molecular Cloning: A Laboratory Manual (Fourth Edition) (http://www.molecularcloning.com), which are incorporated herein in relevant part (possibly in their entirety) by reference.

The present disclosure has been described above with reference to preferred embodiments for easy understanding. Hereinafter, the present disclosure will be described based on examples, but the above description and the following examples are provided for the purpose of illustration only and not for the purpose of limiting the present disclosure. Accordingly, the scope of the present disclosure is not limited to the embodiments or the examples specifically described in the present specification, but is limited only by the claims. Examples

### (Example 1) Method for producing construct

SHIV-NM3rN containing HIV-1 NL432 gene on SIVmac239 background was used as a starting material. Recombinant SHIV was constructed according to previously reported methods (15 and 16). An SHIV-nef vector (SHIV-NI) was constructed from an infectious molecular clone of SHIV-NM3rN (48). A source of the env gene of SHIV-NI was HIV-1 NL432, which is an X4-tropic virus. In SHIV-NI, the nef gene was replaced by unique restriction enzyme sites such as ClaI and ApaI. The Ag85B gene was amplified by PCR using Mycobacterium kansasii as a template and 5'-ATATCGATACCATGTTCTCCCGTCCCGGGCT-3' (ClaI) (SEQ ID NO: 5) and 5'-AGGGCCCCTAGCGGGCGCCCAGGCTGG-3' (ApaI) (SEQ ID NO: 6) primers. Thereafter, the PCR product was digested with restriction enzymes at ClaI and ApaI sites. The plasmid is referred to as pSHIV-Ag85B. SHIV-Ag85B was prepared by transfecting pSHIV-Ag85B into 293 T cells using FuGENE 6 Transfection Reagent (Roche Diagnostics, Indianapolis, IN), and the culture supernatant 48 hours after transfection was stored in liquid nitrogen until use.

### (Example 2) Construct performance confirmation

### (Detection of Ag85B protein)

M8166 cells were infected with SHIV-Ag85B at an MOI of 0.1 and cultured for 1 hour. After washing the cells three times with phosphate buffered saline (PBS), the cells were further cultured in a culture medium for 48 hours. After washing was further performed three times with PBS, the cells were lysed with PBS containing 1.5 M urea, 2% NP-40, and 5% 2-mercaptoethanol. Next, the cells were separated by sodium dodecyl sulfate-polyacrylamide gel electrophoresis, electroblotted to a nitrocellulose membrane, and blocked with 5% non-fat dry milk in PBS containing 0.01% Tween20 (PBST). After washing was further performed three times with PBST, the membrane was incubated with rabbit anti-Ag85B polyclonal antibodies for 2 hours. The membrane was washed three times with NBT/BCIP (Roche Diagnostics, Mannheim, Germany) and then incubated with alkaline phosphatase-labeled anti-rabbit IgG (New England Biolabs, Beverly, MA).

### (In vivo stability of inserted Ag85B gene)

Proviral DNA was extracted from 1 × 10⁶ PBMCs of the inoculated macaque. CD8⁺ depleted PBMCs co-cultured with M8166 cells were also monitored when the virus was re-isolated. Cell DNA was extracted using DNeasy tissue kit (QIAGEN). In order to confirm the stability of the inserted Ag85B gene in SHIV-Ag85B, a proviral DNA fragment containing the inserted Ag85B gene in SHIV-Ag85B was PCR amplified using primers. The sequences of the primers are as described above.

### (Example 3) Evaluation of SHIV-Ag85B therapeutic nucleic acid medicament

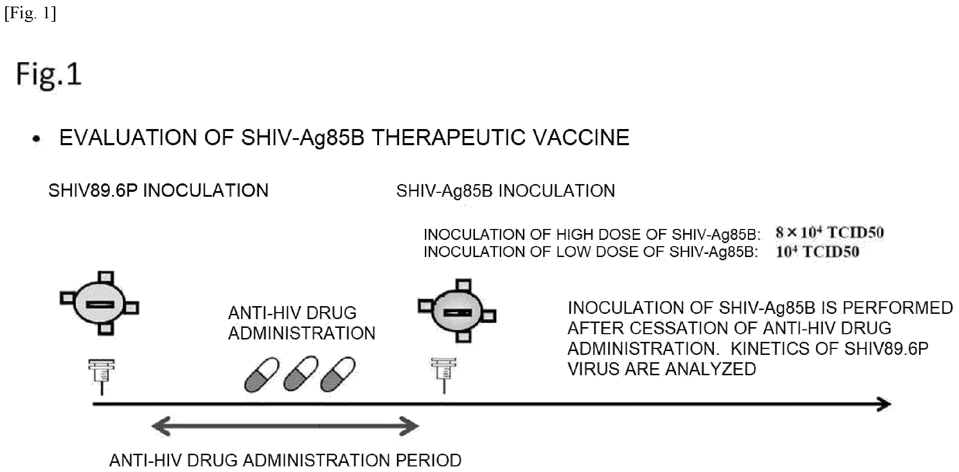

An outline of an experiment of the present example is illustrated in FIG. 1. In the present example, the transitions of the plasma viral loads and the CD4⁺ T cells when the composition according to the present invention was administered after inoculation with the HIV virus were verified.

### (Materials and methods)

### (Viral strains)

In this study, SHIV-Ag85B, SHIV-NI, and SHIV89.6P were used. These viral strains were grown in PBMCs of crab-eating macaques. PBMCs separated by a standard ficoll density gradient separation method were cultured in RPMI1640 to which 10% fetal bovine serum, 2 mM L-glutamine, and 100 units/ml of IL-2 (Shionogi & Co., Ltd.) were added and stimulated with phytohemagglutinin for 72 hours, and cells were infected with SHIV-Ag85B, SHIV-NI, or SHIV89.6P with an infection rate (MOI) of 0.1. Half of the culture medium was replaced with a new culture medium every three days, and a cell-free supernatant was collected from day 6 to day 9 after infection. TCID₅₀ of each SHIV was measured using M8166 cells. TCID₅₀ values of the viral stocks were 5 × 10⁴ in SHIV-Ag85B, 4.7 × 10⁴ in SHIV-NI, and 3 × 10⁵ in SHIV89.6P.

### (Animal)

Adult macaques (from Indonesia, Philippines, and Malaysia) that were all negative for simian SIV, Type D retrovirus, T-cell lymphoma virus, simian foamy virus, Epstein-Barr virus, cytomegalovirus, and B virus were used. Seven macaques were inoculated intravenously with SHIV89.6P50 at 10⁴ TCID₅₀. Thereafter, one week or two weeks after SHIV89.6P infection (after five weeks for #137 only), an anti-HIV drug (tenofovir 20 mg/kg, emtricitabine 40 mg/kg, or dolutegravir 2.5 mg/kg) was subcutaneously administered once a day. After cessation of administration of the anti-HIV drug, five macaques were intravenously inoculated with SHIV-Ag 85B (high dose of SHIV-Ag85B inoculation; 8 × 10⁴ TCID₅₀, low dose of SHIV-Ag85B inoculation; 10⁴ TCID₅₀). Blood was collected periodically using sodium citrate as an anticoagulant and used for measurement of CD4⁺ T cell counts, quantification of plasma viral loads, and immunological analysis. Lymphatic tissue samples were obtained by biopsy and used for measurement of proviral DNA loads and pathological examination.

### (Preparation of DNA sample and amplification of SHIV gag gene by nested PCR)

In order to measure proviral DNA in macaques inoculated with SHIV-Ag85B, nested PCR was used to amplify fragments of the gag gene segments. Proviral DNA was extracted from PBMCs of the inoculated macaques. Cell DNA was extracted using DNeasy tissue kits (QIAGEN). Nested PCR was performed using TaKaRa Ex Taq (Takara Bio Inc., Shiga, Japan). The initial protocol and nested PCR protocol are described in references 49 and 50 (49 and 50). As primers used in this study, Outer SIVgag-F (5'-CCATTAGTGCCAACAGGCTCAG-3' (SEQ ID NO: 7)) and Outer SIVgag-R (5'-CCCCAGTTGGATCCATCTCCTG-3' (SEQ ID NO: 8)) in the first round of PCR, and Nested SIVgag-F (5'-ACTGTCTGCGTCATCTGGTG-3' (SEQ ID NO: 9)) and Nested SIVgag-R (5'-GTCCCAATCTGCAGCCTCCTC-3' (SEQ ID NO: 10)) in the second round of PCR were used. After the second amplification, 10 µl of the product amplified by the nested PCR was applied to a 1.0% agarose gel and stained with ethidium bromide, and DNA bands were visualized. At the first amplification with outer gag primer pairs, the lowest concentration of plasmid SIV DNA detected by the PCR method was 100 copies. Furthermore, single copy plasmid DNA is conventionally detected when amplified with nested/internal gag primers (49 and 50).

### (Viral RNA loads in plasma)

SHIV infection levels were monitored by measuring viral RNA loads in plasma using highly sensitive quantitative real-time RT-PCR as described above (23, 51, 52). Viral RNA was isolated from plasma using MagNA PureCompact Nucleic Acid Isolation Kit (Roche Diagnos-tics). Real-time RT-PCR was performed using QuantiTec Probe RT-PCR Kit (Qiagen) and LightCycler 480 thermocycler (Roche Diagnostics, Rotkreuz, Switzerland). The gag gene of SIVmac239 was amplified with a probe 5'-FAM-TGTCCACCTGCCATTAAGTCCCGA-TAMRA-3' (where FAM is 6-carboxyfluorescein and TAMRA is 6-carboxytetramethylrhodamine) (SEQ ID NO: 11), primers 5'-TGGAAGAAAGACCTCCAGAAAATG-3' (SEQ ID NO: 12) and 5'-CAAGTGCAGTTAGCAAGCGAGGAT-3' (SEQ ID NO: 13). A limit of detection was calculated to be 1,000 viral RNA copies/ml.

### (Proviral DNA loads)

DNA samples were extracted from PBMCs and lymphoid tissue using DNeasy tissue kit (QIAGEN) according to the manufacturer's protocol. Ultra-sensitive digital PCR was performed with the QX200 Droplet Digital PCR system (Bio-Rad). 20 µl of a reaction mixture containing 2 µl of a DNA sample, ddPCR supermix for probes (without dUTP) (Bio-Rad), each of a 900 nM primer and a 200 nM probe, and desalted water was prepared. The mixed solution was placed in a DG8 cartridge with 70 µl of droplet generating oil (Bio-Rad), and droplets were formed in a droplet generator (Bio-Rad). Thereafter, the droplets were transferred to a 96-well microplate. PCR amplification was performed with the following program: initial denaturation and stabilization at 95°C for 10 minutes, denaturation at 94°C for 30 seconds, annealing/extension at 57°C for 60 seconds for 40 cycles, and then, at 98°C for 10 minutes. Thereafter, the droplets were sorted and analyzed with QX200 droplet reader (Bio-Rad) using QuantaSoft v1.6 (Bio-Rad) software. The samples were considered only when 20,000 or more droplets were read. The cell counts were monitored by highly sensitive quantitative real-time PCR as described above (53). DNA samples were extracted from PBMCs and lymphoid tissue using DNADNeasy tissue kit (QIAGEN) according to the manufacturer's protocol. The cell counters were confirmed by detecting cellular IL-4 sequences using rhesus macaque IL-4 specific primers 5'-TGTGCTCCGGCAGTTCTACA-3' (SEQ ID NO: 14) and 5'-CCGTTTCAGGAATCGGATCA-3' (SEQ ID NO: 15) and a probe 5'-FAM-TGCACAGCAGTTCCACAGGCACAAG-TAMRA-3' (SEQ ID NO: 16).

### (CD4⁺ T cell counts)

100 µl of whole blood from each crab-eating macaque was stained with fluorescently labeled monoclonal antibodies: anti-CD3 (Clone SP34-2, Alexa700; BD) and anti-CD4 (Clone L200, PerCP-Cy5.5; BD). Flow cytometry was performed with FACSCanto II flow cytometer (BD). Data were analyzed using FACSDiVa software.

### (Results)

### (Administration of only anti-HIV drug)

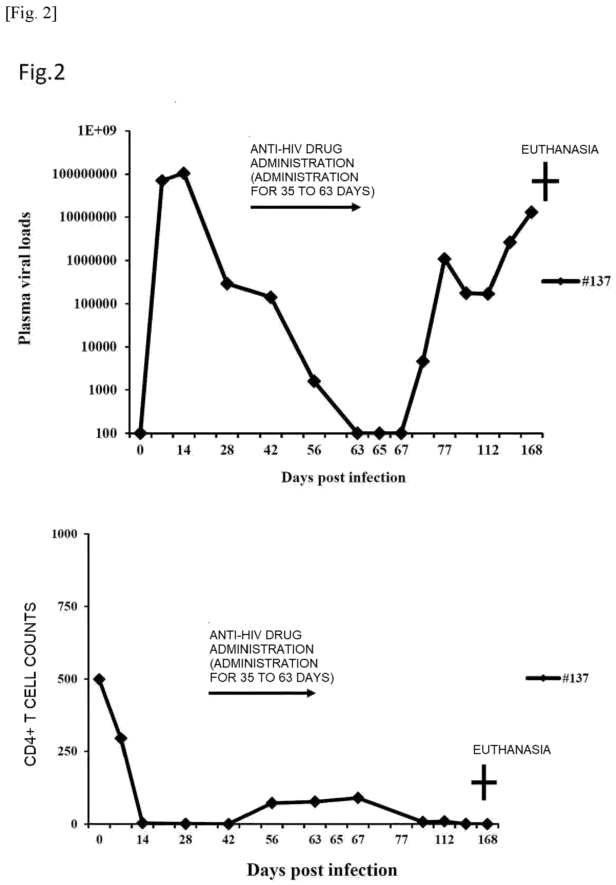

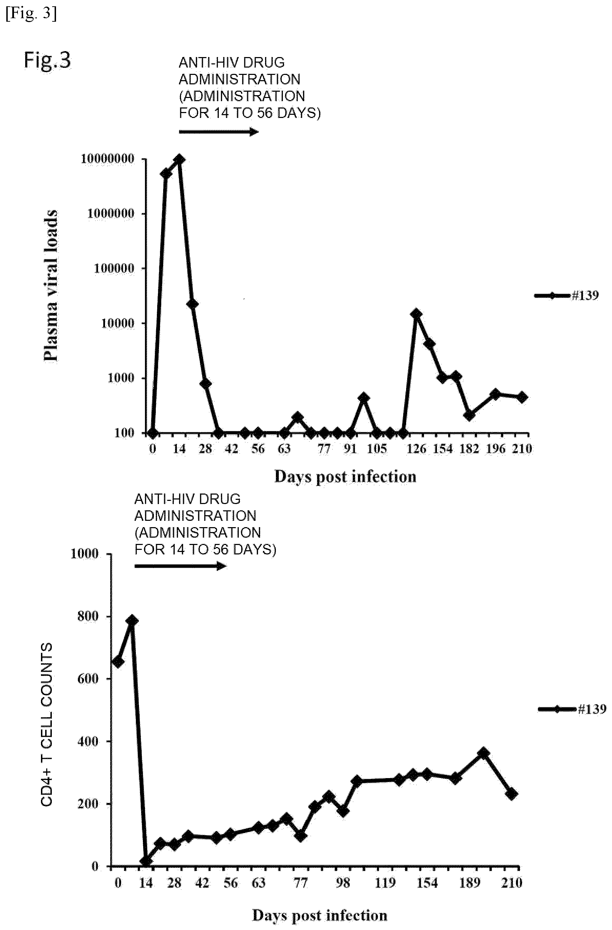

In the macaques (#137 and #139) to which only the anti-HIV drug was administered, plasma viral loads were maintained at low levels, but when the administration of the anti-HIV drug was discontinued, plasma viral loads increased (FIGS. 2 and 3). In addition, in the macaques (#137 and #139) to which SHIV89.6P was inoculated and the anti-HIV drug was administered, CD4⁺ T cell counts were significantly low during the observation period (FIGS. 2 and 3).

### (Two time-administration of low dose of SHIV-Ag85B)

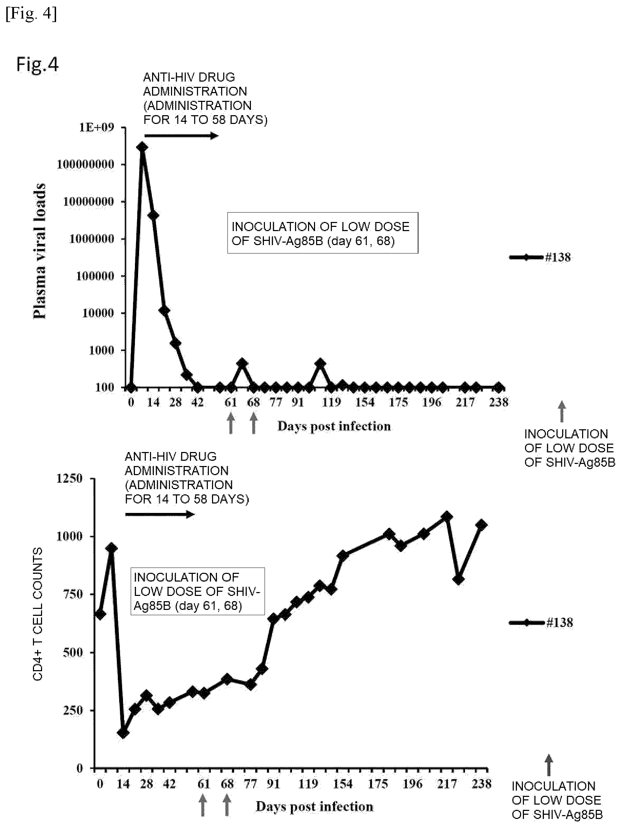

In the macaques (#138) to which a low dose of SHIV- Ag85B was administered two times, almost no virus was detected in plasma even after the administration of the anti-HIV drug was discontinued, and 14 weeks after the first administration of SHIV-Ag85B, the plasma viral loads reached below the limit of detection (FIG. 4). In addition, in the macaques (#138) to which a low dose of SHIV-Ag85B was administered two times, CD4⁺ T cell counts were maintained at normal levels after SHIV-Ag85B administration (FIG. 4).

### (One time-administration of high dose of SHIV-Ag85B)

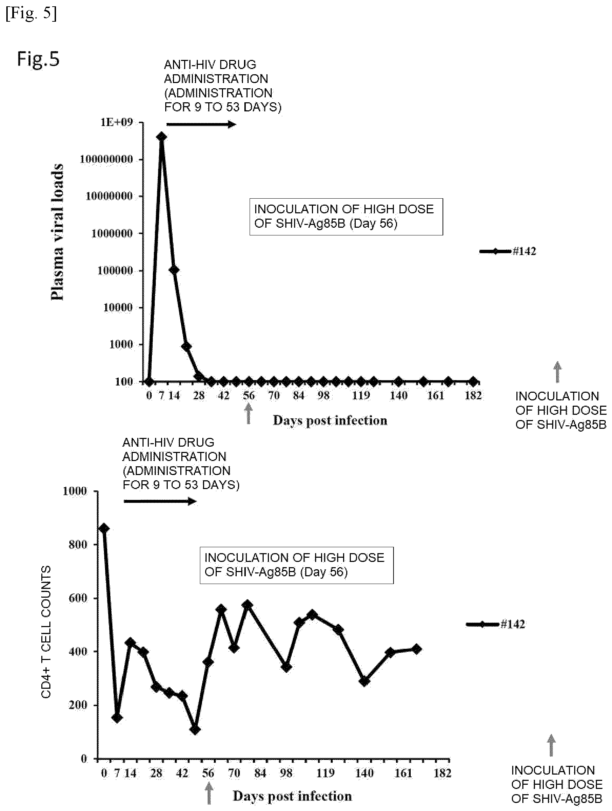

In the macaques (#142) to which a high dose of SHIV-Ag85B was administered once, plasma viral loads were maintained below the limit of detection during the observation period after the administration of the anti-HIV drug was discontinued (FIG. 5). In addition, in the macaques (#142) to which a high dose of SHIV-Ag85B was administered once, CD4⁺ T cell counts were maintained at normal levels after SHIV-Ag85B administration (FIG. 5).

### (Five time-administration of high dose of SHIV-Ag85B)

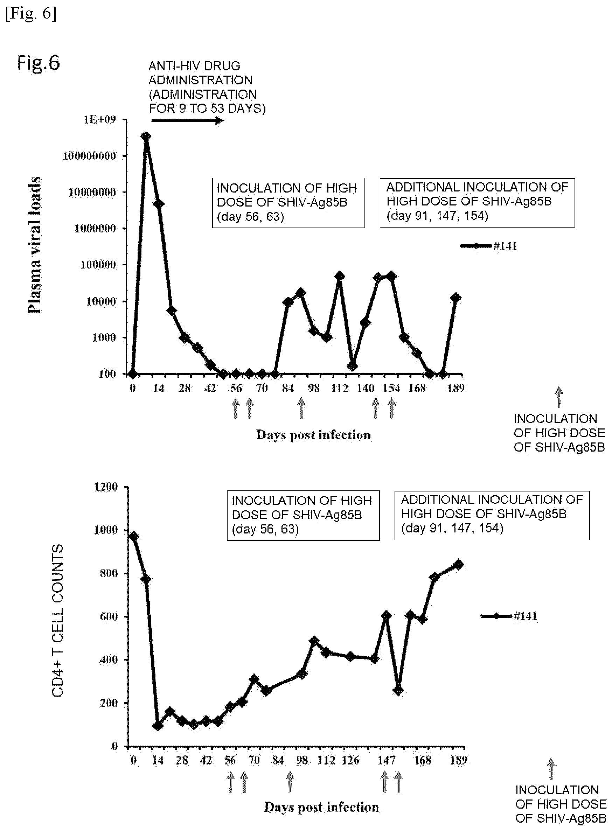

In the macaques (#141) to which a high doses of SHIV-Ag85B was administered, when the high dose of SHIV-Ag85B was administered two times, the plasma viral loads decreased to low levels, but after a while, the plasma viral loads increased (FIG. 6). When a low dose of SHIV-Ag85B was administered again (day 91, day 147, and day 154 after infection), the plasma viral loads decreased. Even in such an example, it is considered that it is possible to maintain the plasma viral loads at low levels by administering SHIV-Ag85B a plurality of times. CD4⁺ T cells were maintained at normal levels (FIG. 6).

### (One time-administration of each of low dose and high dose of SHIV-Ag85B)

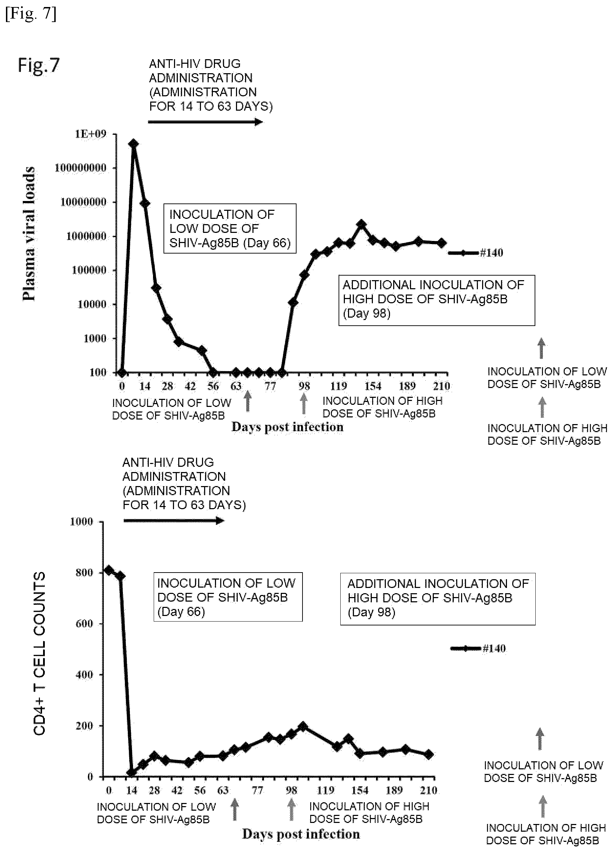

In the macaques (#140) to which a low dose of SHIV-Ag85B was administered on day 66 after infection, the plasma viral loads increased from day 77 after infection. A high dose of SHIV-Ag85B was administered on day 98 after infection, but plasma viral loads were remained increased (FIG. 7). CD4⁺ T cell counts were at low levels during the observation period (FIG. 7).

The experimental method of the present example was performed according to FIG. 1.

The experimental method is specifically as follows.

Start of anti-HIV drug administration:
After infection with SHIV89.6P, plasma viruses peaked (except #137).
Administration method:
Once a day; subcutaneous administration
Anti-HIV drugs:

| | |
|---|---|
| Tenofovir | 20 mg/kg |
| Emtricitabine | 40 mg/kg |
| Dolutegravir | 2.5 mg/kg |

References (Goswami R, et al. Analytical Treatment Interruption after Short-Term Antiretroviral Therapy in a Postnatally Simian-Human Immunodeficiency Virus-Infected Infant Rhesus Macaque Model. mBio. 2019 Sep 5; 10(5): e01971-19. doi: 10.1128/mBio.01971-19., Nishimura Y, et al., Prevention and treatment of SHIVAD8 infection in rhesus macaques by a potent d-peptide HIV entry inhibitor. Proc Natl Acad Sci USA. 2020 Sep 8; 117(36): 22436-22442. doi: 10.1073/pnas.2009700117)

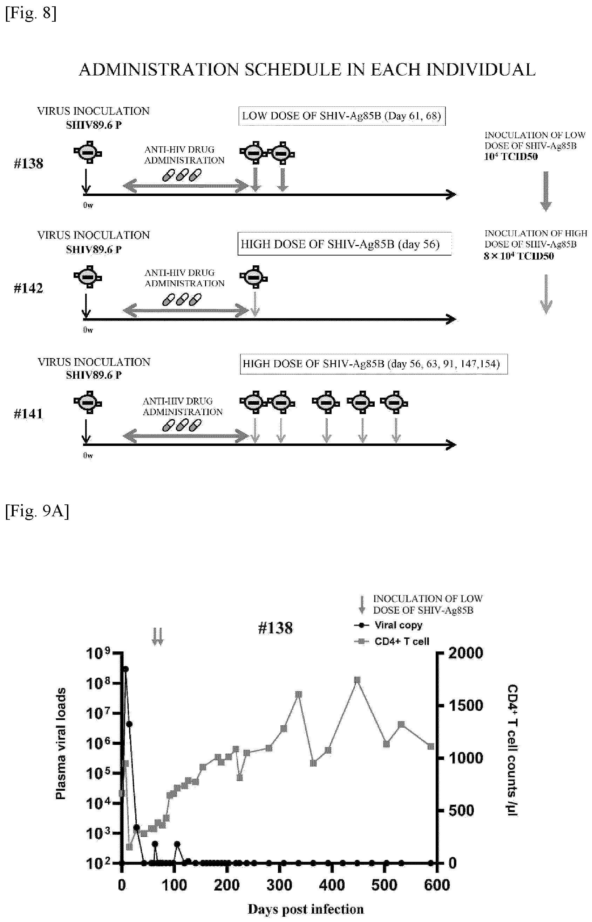

Specifically, it was performed in a procedure substantially similar to the SHIV described above. The administration schedule in each individual is as illustrated in FIG. 8.

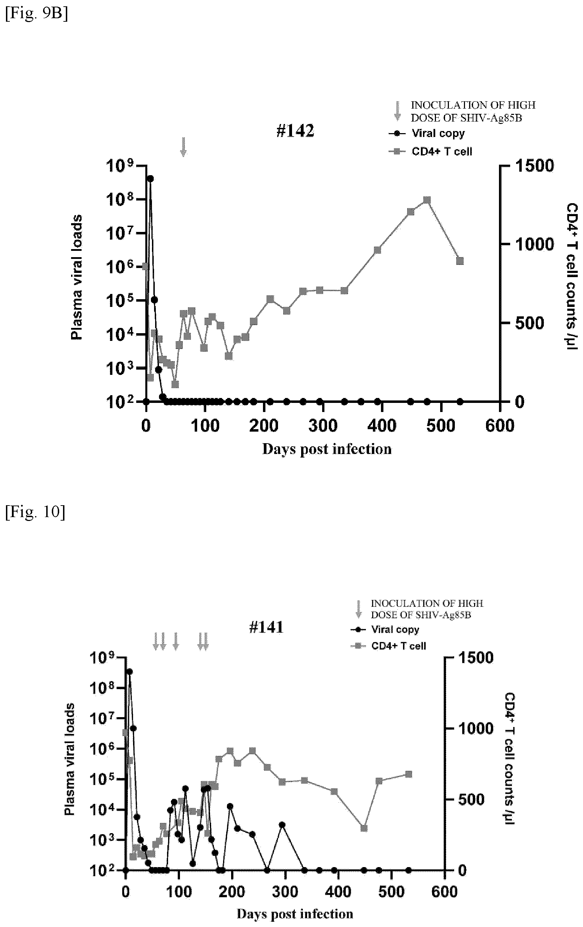

In the macaques (#138) to which a low dose of SHIV- Ag85B was administered two times and the macaques (#142) to which a high dose of SHIV-Ag85B was administered once, plasma viral loads were maintained below the limit of detection for an extended period of time after inoculation with SHIV-Ag85B (FIGS. 9A and 9B). In addition, for one macaque (#141) to which a high dose of SHIV-Ag85B was administered, only a short-term effect was observed in an early stage, and when the SHIV-Ag85B was administered whenever a virus appeared in plasma, a long-term inhibitory effect was observed from the fifth inoculation (FIG. 10).

The above results are described as follows.

In the macaques (#138) to which a low dose of SHIV- Ag85B was administered two times, as the low dose (1 × 10⁴ TCID50) of SHIV-Ag85B was administered on day 4 and day 11 after cessation of the therapeutic drug, the virus in plasma was maintained below the limit of detection, and CD4⁺ cells returned to normal values and maintained.

The above results are described as follows.

In the macaques (#142) to which a high dose of SHIV-Ag85B was administered once, as the high dose (5 × 10⁴ TCID50) of SHIV-Ag85B was administered on day 7 after cessation of the therapeutic drug, the virus in plasma was maintained below the limit of detection, and CD4⁺ cells returned to normal values and maintained.

In the macaques (#141) to which a high dose of SHIV-Ag85B was administered, as the high dose (5 × 10⁴ TCID50) of SHIV-Ag85B was administered on day 7 after cessation of the therapeutic drug, the virus in plasma was maintained below the limit of detection for only a short period of time, and thus, the administration was performed each time the virus appeared in plasma, and a long-term inhibitory effect was observed from the fifth inoculation. CD4⁺ cells also returned to normal values and maintained.

### (Example 4) Additional clinical trials with SHIV-Ag85B

A high dose of SHIV-Ag85B was administered to crab-eating macaques four times every week. Thereafter, the transitions of the plasma viral loads and CD4⁺ T cell counts in the crab-eating macaques were measured.

### (1) Influence of drug cessation

Regarding the influence of drug cessation, the number of times of inoculation and the inoculation amount of SHIV-Ag85B were examined, and the same experiment as in Example 3 was performed. The drug cessation was performed by stopping the drug administration for 3 days after 8 weeks of administration.

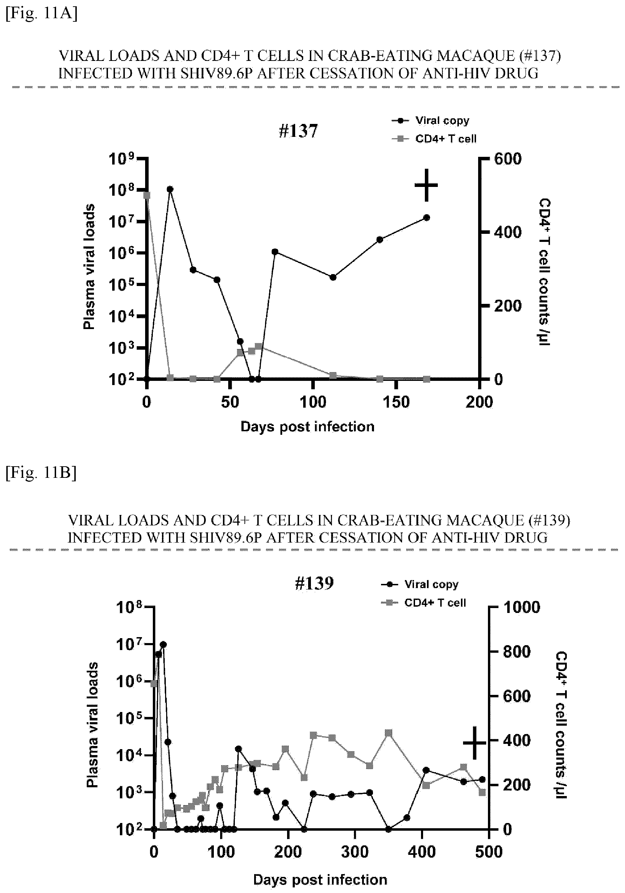

The above results are as illustrated in (FIG. 11A illustrates #137, and FIG. 11B illustrates #139) in FIG. 11.

The above results are described as follows.

FIG. 11A is a control of an untreated macaque (#137) to which only an anti-HIV drug is administered. The virus appeared in plasma on day 7 after drug cessation, and then increased, CD4⁺ cells decreased in an inverse manner, and the macaque died on day 170 after infection.

FIG. 11B is a control of an untreated macaque (#139) to which only an anti-HIV drug is administered. The virus appeared in plasma on day 110 after drug cessation, and then increased, CD4⁺ cells decreased in an inverse manner, and the macaque died on day 500 after infection.

### (2) Optimization of treatment protocol

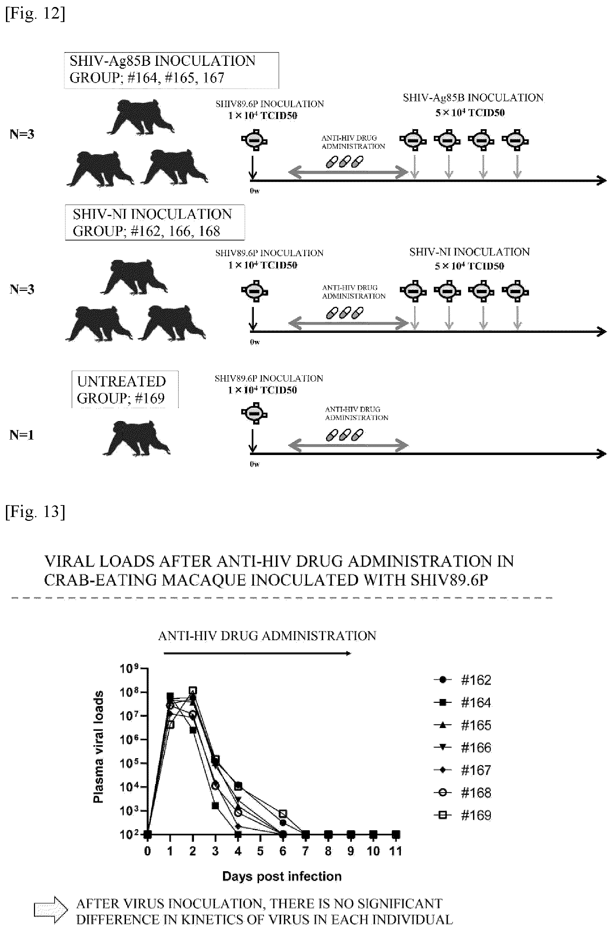

An outline of an experiment of the present example is illustrated in FIG. 12. In the present example, the transition of the plasma viral loads when the composition according to the present disclosure was administered after inoculation with the HIV virus was verified.

### (Protocol)

Administration is started from the first week after SHIV infection. SHIV-Ag85B (5 × 10⁴ TCID50) was administered four times at an interval of one week from day 7 after cessation of the therapeutic drug.

### (Verification method)

### (Viral RNA loads in plasma)

SHIV infection levels were monitored by measuring viral RNA loads in plasma using highly sensitive quantitative real-time RT-PCR as described above (23, 51, 52). Viral RNA was isolated from plasma using MagNA PureCompact Nucleic Acid Isolation Kit (Roche Diagnos-tics). Real-time RT-PCR was performed using QuantiTec Probe RT-PCR Kit (Qiagen) and LightCycler 480 thermocycler (Roche Diagnostics, Rotkreuz, Switzerland). The gag gene of SIVmac239 was amplified with a probe 5'-FAM-TGTCCACCTGCCATTAAGTCCCGA-TAMRA-3' (where FAM is 6-carboxyfluorescein and TAMRA is 6-carboxytetramethylrhodamine) (SEQ ID NO: 11), primers 5'-TGGAAGAAAGACCTCCAGAAAATG-3' (SEQ ID NO: 12) and 5'-CAAGTGCAGTTAGCAAGCGAGGAT-3' (SEQ ID NO: 13). A limit of detection was calculated to be 1,000 viral RNA copies/ml.

### (CD4⁺ T cell counts)

100 µl of whole blood from each crab-eating macaque was stained with fluorescently labeled monoclonal antibodies: anti-CD3 (Clone SP34-2, Alexa700; BD) and anti-CD4 (Clone L200, PerCP-Cy5.5; BD). Flow cytometry was performed with FACSCanto II flow cytometer (BD). Data were analyzed using FACSDiVa software.

### (Results)

The results are illustrated in FIG. 13. The anti-HIV drugs used were shown to be successful regardless of individual differences.

### (Example 5: Treatment optimization)

In the present example, a test in which SHIV-Ag85B was administered four times was performed. The protocol is substantially the same as the protocol described above.

### (Four time-administration of SHIV-Ag85B)

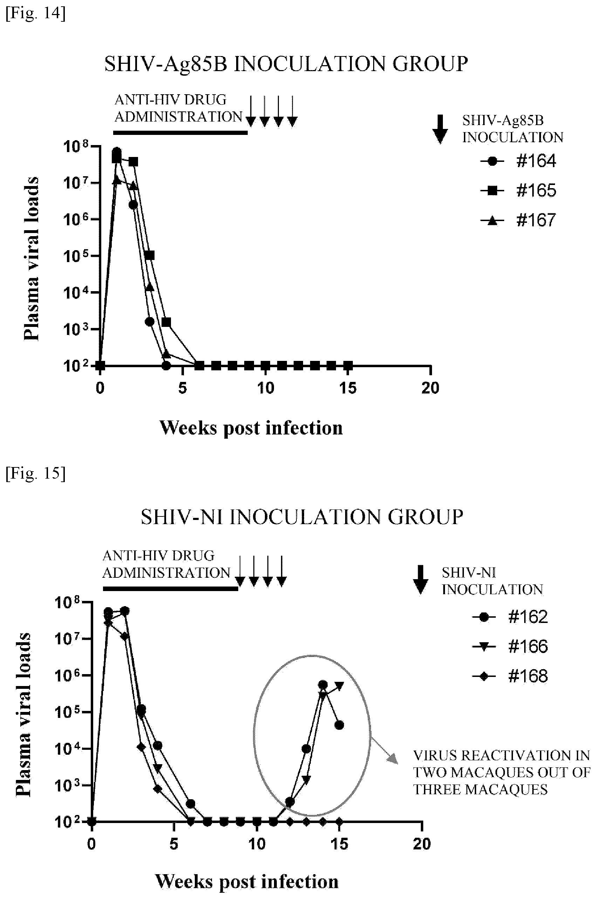

In the macaques (#164, #165, and #167) to which a high dose (5.0 × 10⁴ TCID50) of SHIV-Ag85B was administered, when the high dose of SHIV-Ag85B was administered four times at an interval of one week, the plasma viral loads were maintained below the limit of detection during the observation period (FIG. 14). In the control group containing no Ag85B, the virus appeared in plasma at two weeks after drug cessation, whereas in the SHIV-Ag85B administration group, the virus in plasma was maintained below the limit of detection.

### (Four time-administration of SHIV-NI)

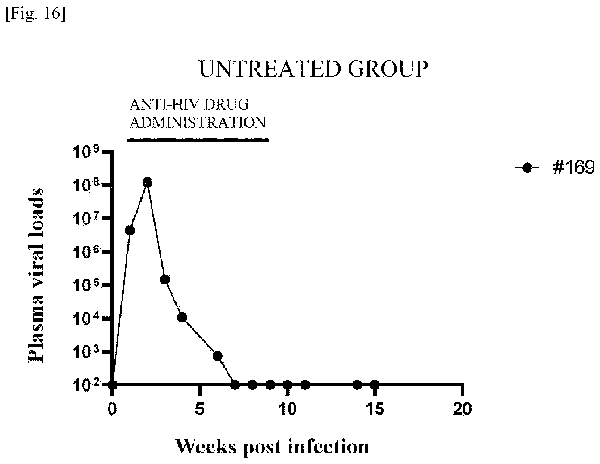

In the macaques (#162, #166, and #168) to which a high dose (5.0 × 10⁴ TCID50) of SHIV-NI was administered, when the high dose of SHIV-NI was administered four times, the plasma viral loads were maintained below the limit of detection in #168, while the plasma viral loads increased in #162 and #166 (FIG. 15). In the control group containing no Ag85B, the virus appeared in plasma at two weeks after drug cessation, whereas in the SHIV-Ag85B administration group, the virus in plasma was maintained below the limit of detection (the control group containing no Ag85B is illustrated in FIG. 15, the SHIV-Ag85B inoculation group is illustrated in FIG. 14, and the untreated group is illustrated in FIG. 16).

### (Example 6) Clinical trials with HIV-Ag85B

In the present example, HIV-Ag85B (SEQ ID NO: 4) is administered subcutaneously or intravenously four to eight times at an interval of one to two weeks after cessation of the anti-HIV therapeutic drug to the HIV infected humans (BCG inoculation group and BCG non-inoculation group) undergoing treatment with the anti-HIV therapeutic drug. The therapeutic effect of HIV-Ag85B is confirmed.

Plasma viral loads and CD4⁺ cell counts are confirmed based on Examples 1 to 5. Since inoculation of a BCG vaccine is expected to enhance the adjuvant effect of Ag85B, the therapeutic effect on HIV by HIV-Ag85B is expected to be high in a BCG inoculation group.

### (Example 7) Formulation examples

When formulation is performed, the formulation can be performed by the following production method.

(Example 1) An appropriate volume of physiological saline can be directly added to an appropriate amount of the lyophilized nucleic acid construct to form an injectable solution formulation.

(Example 2) An appropriate volume of a 5% glucose solution as an isotonic solution can be added to an appropriate amount of the lyophilized nucleic acid construct to form an injectable solution formulation.

(Example 3) To an appropriate amount of the nucleic acid construct dissolved in water, an appropriate volume of 10% Otsuka Saline Injection as an electrolyte correction solution or the like can be added to adjust the concentration to 0.9% NaCl, thereby forming an injectable solution formulation.

(Example 4) An appropriate amount of the nucleic acid construct dissolved in water can be lyophilized to form a lyophilized formulation of the nucleic acid construct sodium salt.

### References

1. Cohen, M. S. et al. Prevention of HIV-1 infection with early antiretroviral therapy. N. Engl. J. Med. 365, 493-505 (2011).
2. Gupta, R. K. et al. HIV-1 remission following CCR5Delta32/Delta32 haematopoietic stem-cell transplantation. Nature 568, 244-248 (2019).
3. Hutter, G. et al. Long-term control of HIV by CCR5 Delta32/Delta32 stem-cell transplantation. N. Engl. J. Med. 360, 692-698 (2009).
4. Burton, D. R. et al. A blueprint for HIV vaccine discovery. Cell Host Microbe 12, 396-407 (2012).
5. Haynes, B. F. et al. HIV-host interactions: implications for vaccine design. Cell Host Microbe 19, 292-303 (2016).
6. Rerks-Ngarm, S. et al. Vaccination with ALVAC and AIDSVAX to prevent HIV-1 infection in Thailand. N. Engl. J. Med. 361, 2209-2220 (2009).
7. Kestler, H. W. 3rd et al. Importance of the nef gene for maintenance of high virus loads and for development of AIDS. Cell 65, 651-662 (1991).
8. Daniel, M. D., Kirchhoff, F., Czajak, S. C., Sehgal, P. K. & Desrosiers, R. C. Protective effects of a live attenuated SIV vaccine with a deletion in the nef gene. Science 258, 1938-1941 (1992).
9. Johnson, R. P. & Desrosiers, R. C. Protective immunity induced by live attenuated simian immunodeficiency virus. Curr. Opin. Immunol. 10, 436-443 (1998).
10. Koff, W. C. et al. HIV vaccine design: insights from live attenuated SIV vaccines. Nat. Immunol. 7, 19-23 (2006).
11. Picker, L. J., Hansen, S. G. & Lifson, J. D. New paradigms for HIV/AIDS vaccine development. Annu. Rev. Med. 63, 95-111 (2012).
12. Baba, T. W. et al. Pathogenicity of live, attenuated SIV after mucosal infection of neonatal macaques. Science 267, 1820-1825 (1995).
13. Baba, T. W. et al. Live attenuated, multiply deleted simian immunodeficiency virus causes AIDS in infant and adult macaques. Nat. Med. 5, 194-203 (1999).
14. Wyand, M. S., Manson, K. H., Lackner, A. A. & Desrosiers, R. C. Resistance of neonatal monkeys to live attenuated vaccine strains of simian immunodeficiency virus. Nat. Med. 3, 32-36 (1997).
15. Shimizu, Y. et al. A genetically engineered live-attenuated simian-human immunodeficiency virus that co-expresses the RANTES gene improves the magnitude of cellular immunity in rhesus macaques. Virology 361, 68-79 (2007).
16. Shimizu, Y. et al. Induction of immune response in macaque monkeys infected with simian-human immunodeficiency virus having the TNF-alpha gene at an early stage of infection. Virology 343, 151-161 (2005).
17. Stahl-Hennig, C. et al. Replication, immunogenicity, and protective properties of live-attenuated simian immunodeficiency viruses expressing interleukin-4 or interferon-gamma. Virology 305, 473-485 (2003).
18. Takamura, S., Matsuo, K., Takebe, Y. & Yasutomi, Y. Ag85B of mycobacteria elicits effective CTL responses through activation of robust Th1 immunity as a novel adjuvant in DNA vaccine. J. Immunol. 175, 2541-2547 (2005).
19. Mori, H. et al. Administration of Ag85B showed therapeutic effects to Th2-type cytokine-mediated acute phase atopic dermatitis by inducing regulatory T cells. Arch. Dermatol. Res. 301, 151-157 (2009).
20. Tsujimura, Y. et al. Effects of mycobacteria major secretion protein, Ag85B, on allergic inflammation in the lung. PLoS ONE 9, e106807 (2014).
21. Tsujimura, Y. & Yasutomi, Y. Allergy vaccines using a mycobacterium-secreted antigen, Ag85B, and an IL-4 antagonist. Methods Mol. Biol. 1403, 723-738 (2016).
22. Watanabe, K. et al. Recombinant Ag85B vaccine by taking advantage of characteristics of human parainfluenza type 2 virus vector showed Mycobacteriaspecific immune responses by intranasal immunization. Vaccine 32, 1727-1735 (2014).
23. Okamura, T. et al. Simian immunodeficiency virus SIVmac239 infection and simian human immunodeficiency virus SHIV89.6P infection result in progression to AIDS in cynomolgus macaques of Asian origin. J. Gen. Virol. 97, 3413-3426 (2016).
24. Alexopoulou, L., Holt, A. C., Medzhitov, R. & Flavell, R. A. Recognition of doublestranded RNA and activation of NF-kappaB by Toll-like receptor 3. Nature 413, 732-738 (2001).
25. Kato, H. et al. Differential roles of MDA5 and RIG-I helicases in the recognition of RNA viruses. Nature 441, 101-105 (2006).
26. Fukazawa, Y. et al. Lymph node T cell responses predict the efficacy of live attenuated SIV vaccines. Nat. Med. 18, 1673-1681 (2012).
27. Reynolds, M. R. et al. Macaques vaccinated with live-attenuated SIV control replication of heterologous virus. J. Exp. Med. 205, 2537-2550 (2008).
28. Villinger, F. et al. Induction of long-term protective effects against heterologous challenge in SIVhu-infected macaques. Virology 278, 194-206 (2000).
29. Giavedoni, L. D., Velasquillo, M. C., Parodi, L. M., Hubbard, G. B. & Hodara, V. L. Expression of IL-18 by SIV does not modify the outcome of the antiviral immune response. Virology 303, 327-337 (2002).
30. Giavedoni, L. D. & Yilma, T. Construction and characterization of replicationcompetent simian immunodeficiency virus vectors that express gamma interferon. J. Virol. 70, 2247-2251 (1996).
31. Berg, R. K. et al. Genomic HIV RNA induces innate immune responses through RIG-I-dependent sensing of secondary-structured RNA. PLoS ONE 7, e29291 (2012).
32. Bosinger, S. E. & Utay, N. S. Type I interferon: understanding its role in HIV pathogenesis and therapy. Curr. HIV/AIDS Rep. 12, 41-53 (2015).
33. Co, J. G., Witwer, K. W., Gama, L., Zink, M. C. & Clements, J. E. Induction of innate immune responses by SIV in vivo and in vitro: differential expression and function of RIG-I and MDA5. J. Infect. Dis. 204, 1104-1114 (2011).
34. Mogensen, T. H., Melchjorsen, J., Larsen, C. S. & Paludan, S. R. Innate immune recognition and activation during HIV infection. Retrovirology 7, 54 (2010).
35. Borducchi, E. N. et al. Ad26/MVA therapeutic vaccination with TLR7 stimulation in SIV-infected rhesus monkeys. Nature 540, 284-287 (2016).
36. Vaccari, M. et al. Adjuvant-dependent innate and adaptive immune signatures of risk of SIVmac251 acquisition. Nat. Med. 22, 762-770 (2016).
37. Goulder, P. J. & Watkins, D. I. Impact of MHC class I diversity on immune control of immunodeficiency virus replication. Nat. Rev. Immunol. 8, 619-630 (2008).
38. Loffredo, J. T. et al. Mamu-B*08-positive macaques control simian immunodeficiency virus replication. J. Virol. 81, 8827-8832 (2007).
39. Muhl, T., Krawczak, M., Ten Haaft, P., Hunsmann, G. & Sauermann, U. MHC class I alleles influence set-point viral load and survival time in simian immunodeficiency virus-infected rhesus monkeys. J. Immunol. 169, 3438-3446 (2002).
40. Yant, L. J. et al. The high-frequency major histocompatibility complex class I allele Mamu-B*17 is associated with control of simian immunodeficiency virus SIVmac239 replication. J. Virol. 80, 5074-5077 (2006).
41. Saito, Y, Naruse, T. K., Akari, H., Matano, T. & Kimura, A. Diversity of MHC class I haplotypes in cynomolgus macaques. Immunogenetics 64, 131-141 (2012).
42. Hansen, S. G. et al. Profound early control of highly pathogenic SIV by an effector memory T-cell vaccine. Nature 473, 523-527 (2011).
43. Hansen, S. G. et al. Effector memory T cell responses are associated with protection of rhesus monkeys from mucosal simian immunodeficiency virus challenge. Nat. Med. 15, 293-299 (2009).
44. Hansen, S. G. et al. Immune clearance of highly pathogenic SIV infection. Nature 502, 100-104 (2013).
45. Martinez-Navio, J. M. et al. Adeno-associated virus delivery of anti-HIV monoclonal antibodies can drive long-term virologic suppression. Immunity 50, 567-575.e565 (2019).
46. Borducchi, E. N. et al. Antibody and TLR7 agonist delay viral rebound in SHIVinfected monkeys. Nature 563, 360-364 (2018).
47. Lim, S. Y. et al. TLR7 agonists induce transient viremia and reduce the viral reservoir in SIV-infected rhesus macaques on antiretroviral therapy. Sci. Transl. Med. 10, eaao4521 (2018).
48. Igarashi, T. et al. Infectivity and immunogenicity of SIVmac/HIV-1 chimeric viruses (SHIVs) with deletions in two or three genes (vpr, nef and vpx). Microbiol. Immunol. 42, 71-74 (1998).
49. Unger, R. E. et al. Detection of simian immunodeficiency virus DNA in macrophages from infected rhesus macaques. J. Med. Primatol. 21, 74-81 (1992).
50. Yoshino, N. et al. Intradermal delivery of recombinant vaccinia virus vector DIs induces gut-mucosal immunity. Scand. J. Immunol. 72, 98-105 (2010).
51. Mori, K. et al. Quintuple deglycosylation mutant of simian immunodeficiency virus SIVmac239 in rhesus macaques: robust primary replication, tightly contained chronic infection, and elicitation of potent immunity against the parental wild-type strain. J. Virol. 75, 4023-4028 (2001).
52. Enose, Y. et al. Protection by intranasal immunization of a nef-deleted, nonpathogenic SHIV against intravaginal challenge with a heterologous pathogenic SHIV. Virology 298, 306-316 (2002).
53. Sugimoto, C. et al. Glycosylation of simian immunodeficiency virus influences immune-tissue targeting during primary infection, leading to immunodeficiency or viral control. J. Virol. 86, 9323-9336 (2012).
54. Amara, R. R. et al. Different patterns of immune responses but similar control of a simian-human immunodeficiency virus 89.6P mucosal challenge by modified vaccinia virus Ankara (MVA) and DNA/MVA vaccines. J. Virol. 76, 7625-7631 (2002).
55. Montefiori, D. C. Measuring HIV neutralization in a luciferase reporter gene assay. Methods Mol. Biol. 485, 395-405 (2009).
56. Pollara, J. et al. Bridging Vaccine-Induced HIV-1 Neutralizing and Effector Antibody Responses in Rabbit and Rhesus Macaque Animal Models. J. Virol. 93, e02119-18 (2019).
57. Yamamoto, T. et al. Virus inhibition activity of effector memory CD8(+) T cells determines simian immunodeficiency virus load in vaccinated monkeys after vaccine breakthrough infection. J. Virol. 86, 5877-5884 (2012).
58. Yamamoto, T. et al. STING agonists activate latently infected cells and enhance SIV-specific responses ex vivo in naturally SIV controlled cynomolgus macaques. Sci. Rep. 9, 5917 (2019).
59. Kuromatsu, I., Matsuo, K., Takamura, S., Kim, G., Takebe, Y, Kawamura, J and Yasutomi, Y. Induction of effective antitumor immune responses by using DNA of an αAg from mycobacteria. Cancer Gene Ther. 2001; 8: 483-490.

### (Note)

As described above, the present disclosure has been illustrated using the preferred embodiments of the present disclosure, but the present disclosure should not be construed as being limited to the embodiments. It is understood that the scope of the present disclosure should be interpreted only by the claims. It is understood from the description of the specific preferred embodiments of the present disclosure that those skilled in the art can implement equivalent ranges based on the description of the present disclosure and the common general knowledge. It is also understood that any patent, any patent application, and any reference cited herein should be incorporated herein by reference in the same manner as the contents are specifically described herein. The present application claims priority to Japanese Patent Application No. 2022-140218 filed on September 2, 2022 to the Japan Patent Office, the entire contents of which are incorporated herein by reference as necessary.

### Industrial Applicability

The present disclosure is useful in the pharmaceutical industry.

### Sequence Listing Free Text

SEQ ID NO: 1: Nucleic acid sequence of Ag85B
SEQ ID NO: 2: Amino acid sequence of Ag85B
SEQ ID NO: 3: Nucleic acid sequence of SHIV-Ag85B
SEQ ID NO: 4: Nucleic acid sequence of HIV-Ag85B
SEQ ID NO: 5: ClaI primer
SEQ ID NO: 6: ApaI primer
SEQ ID NO: 7: Outer SIVgag-F primer
SEQ ID NO: 8: Outer SIVgag-R primer
SEQ ID NO: 9: Nested SIVgag-F primer
SEQ ID NO: 10: Nested SIVgag-R primer
SEQ ID NO: 11: Probe for gag of SIVmac239
SEQ ID NO: 12: Primer 1 for gag of SIVmac239
SEQ ID NO: 13: Primer 2 for gag of SIVmac239
SEQ ID NO: 14: Primer 1 for IL-4
SEQ ID NO: 15: Primer 2 for IL-4
SEQ ID NO: 16: Probe for IL-4

## Claims

1. A composition for treating a viral infection in a subject, the composition comprising a nucleic acid construct containing, in an operable manner, a nucleic acid sequence encoding an antigen protein contained in a virus belonging to the genus Lentivirus or a part thereof and a nucleic acid sequence encoding Ag85B protein.

2. The composition according to claim 1, **characterized in that** the composition is administered after the viral infection.

3. The composition according to claim 2, wherein the treatment for the viral infection substantially eliminates the virus from the subject after the viral infection.

4. The composition according to claim 2, wherein in the treatment for the viral infection, the composition is administered to substantially eliminate the virus from the subject after the viral infection.

5. The composition according to any one of claims 1 to 4, wherein the nucleic acid sequence encoding the antigen protein or part thereof is a nucleic acid sequence encoding an attenuated virus.

6. The composition according to claim 5, wherein the attenuated virus is an nef-deleted attenuated virus.

7. The composition according to claim 5 or 6, wherein the nucleic acid sequence encoding the Ag85B protein is incorporated into the nucleic acid sequence encoding the attenuated virus.

8. The composition according to claim 6, wherein the nucleic acid sequence encoding the Ag85B protein is incorporated at a location of a deleted nef gene in a nucleic acid sequence encoding the nef-deleted attenuated virus.

9. The composition according to any one of claims 1 to 8, wherein the virus is an AIDS virus.

10. The composition according to any one of claims 1 to 9, wherein the virus is an attenuated virus of a virus that infects humans.

11. The composition according to any one of claims 1 to 10, wherein the virus is an attenuated virus of an AIDS virus selected from the group consisting of HIV, SIV, SHIV, and FIV.

12. The composition according to any one of claims 1 to 11, wherein the virus is an attenuated HIV.

13. The composition according to any one of claims 1 to 12, wherein the virus is an nef-deleted attenuated HIV.

14. The composition according to any one of claims 1 to 13, wherein the nucleic acid sequence encoding the antigen protein or part thereof includes a nucleic acid sequence at least 90% identical to a nucleic acid sequence containing nucleotides 1 to 9,633 and 10,612 to 11,022 of SEQ ID NO: 3 or a nucleic acid sequence containing nucleotides 1 to 8,786 and 9,765 to 15,182 of SEQ ID NO: 4.

15. The composition according to any one of claims 1 to 14, wherein the nucleic acid construct contains a nucleic acid sequence at least 90% identical to a nucleic acid sequence set forth in SEQ ID NO: 3 or 4.

16. The composition according to any one of claims 1 to 15, wherein the nucleic acid construct contains a nucleic acid sequence set forth in SEQ ID NO: 3 or 4.

17. The composition according to any one of claims 1 to 16, **characterized in that** the nucleic acid construct is administered once.

18. The composition according to any one of claims 1 to 16, **characterized in that** the nucleic acid construct is administered two or more times.

19. The composition according to claim 18, **characterized in that** the nucleic acid construct is administered once a week, once every two weeks, once every three weeks, once a month, or once every two months.

20. The composition according to any one of claims 1 to 19, **characterized in that** the nucleic acid construct is administered at a dose of 1.0 × 10³ TCID₅₀ or more.

21. The composition according to any one of claims 1 to 20, **characterized in that** the nucleic acid construct is administered at a dose of 1.0 × 10³ TCID₅₀ or more and less than 5.0 × 10⁴ TCID₅₀.

22. The composition according to claim 21, **characterized in that** the nucleic acid construct is administered once a week at a dose of 1.0 × 10³ TCID₅₀ or more and less than 5.0 × 10⁴ TCID₅₀.

23. The composition according to claim 21, **characterized in that** the nucleic acid construct is administered once at a dose of 1.0 × 10³ TCID₅₀ or more and less than 5.0 × 10⁴ TCID₅₀, and when a virus is detected in a body, the nucleic acid construct is further administered.

24. The composition according to any one of claims 1 to 20, **characterized in that** the nucleic acid construct is administered at a dose of 5.0 × 10⁴ TCID₅₀ or more and 5.0 × 10⁶ TCID₅₀ or less.

25. The composition according to claim 24, **characterized in that** the nucleic acid construct is administered once at a dose of 5.0 × 10⁴ TCID₅₀ or more.

26. The composition according to any one of claims 1 to 25, **characterized in that** an anti-HIV drug is already administered to the subject.

27. The composition according to any one of claims 1 to 25, wherein an anti-HIV drug is not yet administered to the subject, or the anti-HIV drug is discontinued at the start of administration of the composition.

28. The composition according to claim 26 or 27, **characterized in that** as the anti-HIV drug, an anti-HIV drug selected from the group consisting of a reverse transcriptase inhibitor, a protease inhibitor, an integrase inhibitor, and a CCR5 inhibitor is administered.

29. The composition according to claim 26 or 27, **characterized in that**, as the anti-HIV drug, an anti-HIV drug selected from the group consisting of tenofovir, emtricitabine, dolutegravir, zidovudine, lamivudine, sanilvudine, didanosine, abacavir, nevirapine, efavirenz, etravirine, rilpivirine, saquinavir, indinavir, nelfinavir, lopinavir/ritonavir combination, atazanavir, fosamprenavir, darunavir, ritonavir, raltegravir, elvitegravir, maraviroc, or any combination thereof is administered.

30. The composition according to any one of claims 1 to 29, wherein the composition completely eliminates the AIDS virus in the subject.

31. The composition according to any one of claims 1 to 30, wherein the composition is not administered before the viral infection.

32. The composition according to any one of claims 1 to 31, wherein the subject is previously inoculated with a BCG vaccine.
